# EUROPEAN PATENT APPLICATION

(11) **EP 3 048 116 A1**
(43) Date of publication of application: **27.07.2016**
(21) Application number: 15305079.4
(22) Date of filing: 23.01.2015
(51) Int. Cl.: C07K 16/28, A61K 39/395, A61P 35/00, A61K 39/00

(54) **ANTI-DR5 ANTIBODIES WITH ENHANCED APOPTOSIS POTENCY**

(71) Applicant: International-Drug-Development-Biotech, 69007 Lyon (FR)
(72) Inventor: Vermot-Desroches, Claudine, 69570 Dardilly (FR); Vuillermoz, Boris Sébastien, 69380 Les Cheres (FR)
(74) Representative: Colombet, Alain André

(57) **Abstract**

The present disclosure relates to binding specifically to the DR5 receptor and that comprise specific CDRs and variations at positions 326, 333 and 396 (Fc region) which may provide to regulate apoptosis potency. The variants have no modified glycan profiles. The present disclosure claims pharmaceutical compositions and the treatment of cancers, autoimmune disease, inflammatory disease or condition, infectious disease, e.g. viral infection and viral disease, neurological disorder.

SEQUENCE LISTING

<110>  IDD BIOTECH (INTERNATIONAL-DRUG-DEVELOPMENT BIOTECH)

<120>  ANTI-DR5 ANTIBODIES WITH ENHANCED APOPTOSIS POTENCY

<130>  BET14L2417

<160>  33

<170>  PatentIn version 3.5

<210>  1
<211>  354
<212>  DNA
<213>  Artificial Sequence

<220>
<223>  Hz033 VH

<400>  1
gaggtgcaac tgcagcaatc aggagcagaa gtcgtaaagc ccggtgcctc cgttaaactt      60

agctgcaaag caagtgggtt taatatcaaa gatacattca tccattgggt caaacaggca     120

ccaggccagg gccttgaatg gatcggtcgc atcgacccag ctaacggaaa cactaagtat     180

gaccctaagt tccagggaaa ggctacaatt acaacagata catcctccaa taccgcttac     240

atggagctgt cttccttgcg gtctgaggat actgctgtgt attactgtgt acgtgggctg     300

tacacatatt acttcgatta ttggggccag gggactcttg taaccgtttc ctcc           354


<210>  2
<211>  120
<212>  PRT
<213>  Artificial Sequence

<220>
<223>  Hz033 VH

<400>  2

His Glx Glu Val Gln Leu Gln Gln Ser Gly Ala Glu Val Val Lys Pro
1               5                   10                  15


Gly Ala Ser Val Lys Leu Ser Cys Lys Ala Ser Gly Phe Asn Ile Lys
            20                  25                  30


Asp Thr Phe Ile His Trp Val Lys Gln Ala Pro Gly Gln Gly Leu Glu
        35                  40                  45


Trp Ile Gly Arg Ile Asp Pro Ala Asn Gly Asn Thr Lys Tyr Asp Pro
    50                  55                  60


Lys Phe Gln Gly Lys Ala Thr Ile Thr Thr Asp Thr Ser Ser Asn Thr
65                  70                  75                  80

Ala Tyr Met Glu Leu Ser Ser Leu Arg Ser Glu Asp Thr Ala Val Tyr
                85                  90                  95

Tyr Cys Val Arg Gly Leu Tyr Thr Tyr Tyr Phe Asp Tyr Trp Gly Gln
            100                 105                 110

Gly Thr Leu Val Thr Val Ser Ser
        115                 120

<210>  3
<211>  321
<212>  DNA
<213>  Artificial Sequence

<220>
<223>  Hz033 VL

<400>  3
gagattgtta tgacacagtc ccctgctaca ttgagtgtta gtccaggcga aagagctaca        60

ctgtcatgta gggcctctca gtctattagc aataacctgc actggtatca gcaaaagcca       120

ggtcaagccc ccaggctgct gattaagttt gcatctcaaa gtattacagg aatccctgct       180

cggttcagcg gctccgggag tggaaccgag tttacactca caatctcaag cctccagtcc       240

gaagacttcg ccgtatatta ctgtcagcag ggcaactctt ggccctacac cttcggtcag       300

ggaaccaagc tggagatcaa g                                                 321

<210>  4
<211>  107
<212>  PRT
<213>  Artificial Sequence

<220>
<223>  Hz033 VL

<400>  4

Glu Ile Val Met Thr Gln Ser Pro Ala Thr Leu Ser Val Ser Pro Gly
1               5                   10                  15

Glu Arg Ala Thr Leu Ser Cys Arg Ala Ser Gln Ser Ile Ser Asn Asn
            20                  25                  30

Leu His Trp Tyr Gln Gln Lys Pro Gly Gln Ala Pro Arg Leu Leu Ile
        35                  40                  45

Lys Phe Ala Ser Gln Ser Ile Thr Gly Ile Pro Ala Arg Phe Ser Gly
    50                  55                  60

Ser Gly Ser Gly Thr Glu Phe Thr Leu Thr Ile Ser Ser Leu Gln Ser
65                  70                  75                  80

Glu Asp Phe Ala Val Tyr Tyr Cys Gln Gln Gly Asn Ser Trp Pro Tyr
                85                  90                  95

Thr Phe Gly Gln Gly Thr Lys Leu Glu Ile Lys
            100                 105

<210>  5
<211>  354
<212>  DNA
<213>  Artificial Sequence

<220>
<223>  Hz031 VH

<400>  5
caagttcagt tggtacaatc aggtgcagaa gttaaaaagc caggggcatc tgttaaagtg      60

tcttgcaagg cctccggctt taatatcaag gacacacaca tgcattgggt gcgccaggcc     120

ccaggccagc gactggagtg gattgggcgt attgaccccg ccaacggcaa caccgagtat     180

gaccagaagt ttcagggccg tgtaaccatc accgtcgata cctcagcatc aaccgcttac     240

atggagcttt catctcttcg gtccgaagac acagccgtct attactgcgc tcgatgggga     300

acaaacgttt actttgcata ttggggtcag ggtactctcg tcaccgtgag cagt           354

<210>  6
<211>  122
<212>  PRT
<213>  Artificial Sequence

<220>
<223>  Hz031 VH

<400>  6

Ala Met Ile Asn Gln Val Gln Leu Val Gln Ser Gly Ala Glu Val Lys
1               5                   10                  15

Lys Pro Gly Ala Ser Val Lys Val Ser Cys Lys Ala Ser Gly Phe Asn
            20                  25                  30

Ile Lys Asp Thr His Met His Trp Val Arg Gln Ala Pro Gly Gln Arg
        35                  40                  45

Leu Glu Trp Ile Gly Arg Ile Asp Pro Ala Asn Gly Asn Thr Glu Tyr
    50                  55                  60

Asp Gln Lys Phe Gln Gly Arg Val Thr Ile Thr Val Asp Thr Ser Ala
65                  70                  75                  80

Ser Thr Ala Tyr Met Glu Leu Ser Ser Leu Arg Ser Glu Asp Thr Ala

85                  90                  95

Val Tyr Tyr Cys Ala Arg Trp Gly Thr Asn Val Tyr Phe Ala Tyr Trp
            100                 105                 110

Gly Gln Gly Thr Leu Val Thr Val Ser Ser
        115                 120

<210>  7
<211>  318
<212>  DNA
<213>  Artificial Sequence

<220>
<223>  Hz031 VL

<400>  7
gatattcaac ttacacaatc tccctcttct ctctccgctt cagtagggga tagagtgacc      60

atcacttgct ctgcttcttc cagcgtgtca tatatgtatt ggtatcagca aaagcccggc     120

aaggcaccta aaccatggat ttacagaacc agcaatcttg ccagcggtgt tccaagtagg     180

tttagcggct ccggctctgg tacagacttt accctgacta tctcctctct ccagcccgag     240

gattttgcca catattactg ccagcaatac cattcttacc ctccaacttt tggaggtggc     300

actaaggtgg agatcaag                                                   318

<210>  8
<211>  106
<212>  PRT
<213>  Artificial Sequence

<220>
<223>  Hz031 VL

<400>  8

Asp Ile Gln Leu Thr Gln Ser Pro Ser Ser Leu Ser Ala Ser Val Gly
1               5                   10                  15

Asp Arg Val Thr Ile Thr Cys Ser Ala Ser Ser Ser Val Ser Tyr Met
            20                  25                  30

Tyr Trp Tyr Gln Gln Lys Pro Gly Lys Ala Pro Lys Pro Trp Ile Tyr
        35                  40                  45

Arg Thr Ser Asn Leu Ala Ser Gly Val Pro Ser Arg Phe Ser Gly Ser
    50                  55                  60

Gly Ser Gly Thr Asp Phe Thr Leu Thr Ile Ser Ser Leu Gln Pro Glu
65                  70                  75                  80

Asp Phe Ala Thr Tyr Tyr Cys Gln Gln Tyr His Ser Tyr Pro Pro Thr
                85                  90                  95

Phe Gly Gly Gly Thr Lys Val Glu Ile Lys
            100                 105

<210>  9
<211>  993
<212>  DNA
<213>  Homo sapiens

<400>  9
gcctccacca agggcccatc ggtcttcccc ctggcaccct cctccaagag cacctctggg      60

ggcacagcgg ccctgggctg cctggtcaag gactacttcc ccgaaccggt gacggtgtcg     120

tggaactcag gcgccctgac cagcggcgtg cacaccttcc cggctgtcct acagtcctca     180

ggactctact ccctcagcag cgtcgtgacc gtgccctcca gcagcttggg cacccagacc     240

tacatctgca acgtgaatca caagcccagc aacaccaagg tggacaagaa agttgagccc     300

aaatcttgtg acaaaactca cacatgccca ccgtgcccag cacctgaact cctgggggga     360

ccgtcagtct tcctcttccc cccaaaaccc aaggacaccc tcatgatctc ccggacccct     420

gaggtcacat gcgtggtggt ggacgtgagc cacgaagacc ctgaggtcaa gttcaactgg     480

tacgtggacg gcgtggaggt gcataatgcc aagacaaagc cgcgggagga gcagtacaac     540

agcacgtacc gtgtggtcag cgtcctcacc gtcctgcacc aggactggct gaatggcaag     600

gagtacaagt gcaaggtctc caacaaagcc ctcccagccc ccatcgagaa aaccatctcc     660

aaagccaaag ggcagccccg agaaccacag gtgtacaccc tgcccccatc ccgggatgag     720

ctgaccaaga accaggtcag cctgacctgc ctggtcaaag gcttctatcc cagcgacatc     780

gccgtggagt gggagagcaa tgggcagccg gagaacaact acaagaccac gcctcccgtg     840

ctggactccg acggctcctt cttcctctac agcaagctca ccgtggacaa gagcaggtgg     900

cagcagggga acgtcttctc atgctccgtg atgcatgagg ctctgcacaa ccactacacg     960

cagaagagcc tctccctgtc tccgggtaaa tag                                  993

<210>  10
<211>  330
<212>  PRT
<213>  Homo sapiens

<400>  10

<210>  11
<211>  324
<212>  DNA
<213>  Homo sapiens

<400>  11
cgaactgtgg ctgcaccatc tgtcttcatc ttcccgccat ctgatgagca gttgaaatct      60

ggaactgcct ctgttgtgtg cctgctgaat aacttctatc ccagagaggc caaagtacag     120

tggaaggtgg ataacgccct ccaatcgggt aactcccagg agagtgtcac agagcaggac     180

agcaaggaca gcacctacag cctcagcagc accctgacgc tgagcaaagc agactacgag     240

aaacacaaag tctacgcctg cgaagtcacc catcagggcc tgagctcgcc cgtcacaaag     300

agcttcaaca ggggagagtg ttag                                            324

<210>  12
<211>  107
<212>  PRT
<213>  Homo sapiens

<400>  12

<210>  13
<211>  8
<212>  PRT
<213>  Mus musculus

<400>  13

Gly Phe Asn Ile Lys Asp Thr Phe
1               5

<210>  14
<211>  8
<212>  PRT
<213>  Mus musculus

<400>  14

Ile Asp Pro Ala Asn Gly Asn Thr
1               5

<210>  15
<211>  11
<212>  PRT
<213>  Mus musculus

<400>  15

Val Arg Gly Leu Tyr Thr Tyr Tyr Phe Asp Tyr
1               5                   10

<210>  16
<211>  6
<212>  PRT
<213>  Mus musculus

<400>  16

Gln Ser Ile Ser Asn Asn
1               5

<210>  17
<211>  9
<212>  PRT
<213>  Mus musculus

<400>  17

Gln Gln Gly Asn Ser Trp Pro Tyr Thr
1               5

<210>  18
<211>  8
<212>  PRT

<213>  Mus musculus

<400>  18

Gly Phe Asn Ile Lys Asp Thr His
1               5


<210>  19
<211>  12
<212>  PRT
<213>  Mus musculus

<400>  19

Asp Ala Arg Trp Gly Thr Asn Val Tyr Phe Ala Tyr
1               5                   10


<210>  20
<211>  5
<212>  PRT
<213>  Mus musculus

<400>  20

Ser Ser Val Ser Tyr
1               5


<210>  21
<211>  13
<212>  PRT
<213>  Mus musculus

<400>  21

Gln Gln Tyr His Ser Tyr Pro Pro Thr Asp Arg His Val
1               5                   10


<210>  22
<211>  5
<212>  PRT
<213>  Mus musculus

<400>  22

Asp Thr Phe Ile His
1               5


<210>  23
<211>  17
<212>  PRT
<213>  Mus musculus

<400>  23

Arg Ile Asp Pro Ala Asn Gly Asn Thr Lys Tyr Asp Pro Lys Phe Gln
1               5                   10                  15

Gly

<210> 24
<211> 9
<212> PRT
<213> Mus musculus

<400> 24

Gly Leu Tyr Thr Tyr Tyr Phe Asp Tyr
1               5

<210> 25
<211> 11
<212> PRT
<213> Mus musculus

<400> 25

Arg Ala Ser Gln Ser Ile Ser Asn Asn Leu His
1               5                   10

<210> 26
<211> 7
<212> PRT
<213> Mus musculus

<400> 26

Phe Ala Ser Gln Ser Ile Ser
1               5

<210> 27
<211> 5
<212> PRT
<213> Mus musculus

<400> 27

Asp Thr His Ile His
1               5

<210> 28
<211> 17
<212> PRT
<213> Mus musculus

<400> 28

Arg Ile Asp Pro Ala Asn Gly Asn Thr Glu Tyr Asp Pro Lys Phe Gln
1               5                   10                  15

Gly

<210>  29
<211>  9
<212>  PRT
<213>  Mus musculus

<400>  29

Trp Gly Thr Asn Val Tyr Phe Ala Tyr
1               5


<210>  30
<211>  10
<212>  PRT
<213>  Mus musculus

<400>  30

Ser Ala Ser Ser Ser Val Ser Tyr Met Tyr
1               5                   10


<210>  31
<211>  7
<212>  PRT
<213>  Mus musculus

<400>  31

Arg Thr Ser Asn Leu Ala Ser
1               5


<210>  32
<211>  4
<212>  PRT
<213>  Mus musculus

<400>  32

Lys Asp Thr Phe
1


<210>  33
<211>  4
<212>  PRT
<213>  Mus musculus

<400>  33

Lys Asp Thr His
1

## Description

### FIELD OF THE INVENTION

The present invention relates to novel polypeptides binding specifically to the DR5 receptor also called TRAIL receptor 2. The present invention relates to novel monoclonal antibody (MAb) variants that comprise at least 2 or 3 novel or more amino residues which may provide to regulate apoptosis potency. Preferably, these MAb variants have no altered binding to the target. In a first embodiment, with respect to the same antibody having a natural Fc region (the native or wild-type antibody), this invention provides MAb variants comprising novel amino residues that has no modified glycan profiles, no altered complemented dependent cytotoxicity (CDC) activity and/or no altered antibody-dependent cell mediated cytotoxicity (ADCC) and/or no altered MAb affinity. In a specific embodiment, the antibodies may include additional mutations in the Fc region and have enhanced ADCC and/or modulate CDC. In this embodiment, the antibody may have a different glycan profile. The antibody of the invention allows to reverse apoptosis resistance of highly apoptosis resistant DR5 expressing cells. The invention further provides methods and protocols for the applications of said MAb variants, particularly for therapeutic purposes. The invention also relates to pharmaceutical compositions containing these polypeptides or MAb and the treatment of various diseases, especially cancer, autoimmune disease, inflammatory disease or condition, infectious disease, e.g. viral infection and viral disease, neurological disorder.

### BACKGROUND OF THE INVENTION

There is a significant need for cancer, autoimmune or inflammation treatments, particularly for treatment of cancer that has proved refractory to standard cancer treatments, such as surgery, radiation therapy, chemotherapy, and hormonal therapy. A promising alternative is immunotherapy, in which cancer cells are specifically targeted by cancer antigen-specific antibodies.

Recently, therapeutic antibodies have been shown to improve overall survival as well as time to disease progression more particularly in a variety of human malignancies, such as breast, colon and haematological cancers.

Apoptosis, or programmed cell death, is a physiologic process essential to the normal development and homeostasis of multicellular organisms. Derangements of apoptosis contribute to the pathogenesis of several human diseases including cancer, neurodegenerative disorders, and acquired immune deficiency syndrome.

The tumor necrosis factor (TNF)-related apoptosis-inducing ligand (TRAIL), a member of the TNF superfamily of cytokines, is a type 2 membrane protein that is expressed in the majority of normal tissues and can undergo protease cleavage, resulting in a soluble form able to bind to TRAIL receptors, *(*Wiley SR. et al., Immunity. 1995; 3:673-682*;* Daniel PT et al., J Immunol. 1994; 152:5624*).*

Ligands of this family generally recognize and bind to a limited subset of cognate receptors on the cell surface, leading to signal transduction cascades downstream of the receptor, allowing the activation of a large panel of signaling pathway including NF-kB or caspase activation. TRAIL induces apoptosis of certain transformed cells, including a number of different types of cancer cells as well as virally infected cells, while not inducing apoptosis of a number of normal cell types and is thus of particular interest in the development of cancer therapies, *(*Walczak et al., Nature Medecine. 1999; 5/157-163*,* Ashkenazi A. et al., J Clin Invest. 1999; 104:155*).*

There are four known cell surface receptors for TRAIL. TRAIL Receptor 1 (TRAIL-R1, DR4) and Trail Receptor 2 (TRAIL-R2, DR5, Apo-2, TRICK2, Killer, TR6, and Tango-63) have a cytoplasmic death domain and are able to trigger apoptosis in tumor cells via downstream caspase activation. The other two receptors TRAIL Receptor 3 (TRAIL-R3, DcR1, TR5, TRIDD, LIT) and TRAIL Receptor 4 (TRAIL-R4, DcR2, TRUNDD) lack a cytoplasmic death domain and do not mediate apoptosis. In addition, osteoprotegerin (OPG), a soluble (secreted) member of the TNF receptor family of proteins, also binds TRAIL.

The intracytoplasmic domains of DR5 includes a so-called death domain. After activation of its receptors DR5 the fas-associated death domain adapter molecule is recruited to the receptor, leading to an autoproteolytic cleavage and activation of initiator caspase-8. DR5 has been reported to transduce an apoptotic signal to TRAIL sensitive cancer cells, upon binding of TRAIL. Active caspase-8 in turn triggers the proteolytic activation of downstream caspases including caspase-3. Downstream caspases ultimately degrade a broad range of cellular proteins, and apoptosis is finalized.

Expression of DR5 is frequently detected in human cancers, including colon, gastric, pancreatic, ovarian, breast, and non-small-cell lung cancer with low or no expression in normal tissues.

In the development or progression of many diseases it is often the case that cells are not deleted. In many autoimmune diseases and inflammatory conditions, the surviving activated cells attack normal tissues or cells. Further, progression of tumorigenesis and the proliferative panus formation of rheumatoid arthritis are characterized by the unchecked proliferation of cells. Thus insufficient apoptosis leads to the development of disease, and the uses of apoptosis-inducing ligand or agonistic MAb to enhance apoptosis are considered as a potential therapeutic strategy for eliminating those unwanted cells

Agonistic antibodies against DR5 have been produced and represent a new generation of cancer therapy. Works have been conducted also on the use of such agonistic antibodies in order to induce the TRAIL apoptotic pathway.

Agonistic monoclonal antibodies that specifically bind to DR5 are supposed to be able to directly induce apoptosis of targeted tumor cells, *(*Buchsbaum DJ et al., Future Oncol. 2006; 2:493; Rowinsky EK et al., J Clin Oncol. 2005; 23:9394*).*

These include anti-DR5 MAb lexatumumab, *(*Plummer R. et al., Clin Cancer Res. 2007; 13:6187*),* the anti-DR5 MAb apomab, *(*Adams C. et al., Cell Death Differ. 2008; 15:751*),* the anti-DR5 MAb LBy135, *(*Li J. et al., AACR Meeting Abstracts. 2007. Abstract 4874*),* the anti-DR5 MAb WD-1, *(*Wang J. et al., Cell Mol Immunol. 2008; 5:55*)* and the anti-DR5 MAb AMG655, *(*Wall J. et al., AACR Meeting Abstracts. 2008. Abstract 1326*,* Kaplan-Lefko P. et al., AACR Meeting Abstracts. 2008. Abstract 399*).*

Lexatumumab (also known as ETR2-ST01) is an agonistic human monoclonal antibody against DR5 used in the treatment of cancer. HGS-ETR2 antibodies were generated by HGS through collaboration with Cambridge Antibody Technology.

Tigatuzumab (CS-1008) is a humanized IgG1 monoclonal antibody composed of the CDR regions of mTRA-8. The murine anti-DR5 monoclonal antibody, TRA-8 (mTRA8), was selected from a series of anti-DR5 monoclonal antibodies based on its specificity, ability to trigger apoptosis *in vitro* without the use of crosslinking reagents, and lack of toxicity to human hepatocytes, *(*Buchsbaum DJ et al., Clin Cancer Res. 2003; 9:3731*;* Ichikawa K. et al., Nat Med. 2001; 7:954*).*

Tigatuzumab mediates a very similar pattern of *in vitro* cytotoxicity and *in vivo* antitumor efficacy as mTRA-8. It was shown to have potent *in vitro* cytotoxicity to a variety of human tumor cell lines and *in vivo* antitumor efficacy in murine xenograft models of human cancers. Its *in vitro* cytotoxicity and *in vivo* antitumor efficacy can be substantially enhanced in combination with a variety of chemotherapeutic agents and/or radiation *(*Buchsbaum DJ et al., Clin Cancer Res. 2003; 9:3731*;* DeRosier LC et al., Clin Cancer Res. 2007; 13:5535s*).*

An objective of the invention is to provide DR5-binding antibodies that are agonists of the apoptotic function of DR5. More particularly, the objective is to provide for apoptosis improvement of existing antibodies and/or to provide antibodies able to induce apoptosis of DR5 expressing cells that are naturally resistant or little sensitive to apoptosis in response to usual anti-DR5 antibodies, and/or for a manner of designing new DR5-binding (or anti-DR5) antibodies having strong apoptosis potency.

Another objective of the invention is to provide for such antibodies that have a controlled ADCC and/or CDC. In particular, the objective is to provide for apoptosis potency increase with no ADCC induction, no CDC modulation and no glycan profile impact. More particularly, the objective is to provide for apoptosis improvement of existing antibodies without significant alteration of ADCC, CDC and glycan profile of the native or wild-type antibody. Preferably, an objective is also not to alter the MAb affinity and/or the glycan profile with respect to the native or wild-type antibody.

### SUMMARY OF THE INVENTION

The present inventors have now found that unexpectedly, it is possible to enhance apoptosis potency of a DR5-binding antibody, though directed mutagenesis of the Fc region of said antibody. Apoptosis potency of a DR5-binding antibody means apoptosis of sensitive DR5-expressing cells, especially transformed DR5-expressing cells as those found in some cancers and infected cells, as well as DR5 expressing cells that are naturally resistant to apoptosis in response to usual anti-DR5 antibodies. The inventors have also found that it is possible to enhance this valuable property while controlling ADCC and CDC functions. In particular, the inventors have found how enhancing the apoptosis potency without induction or enhancing or significantly enhancing ADCC and/or CDC and/or without dramatically changing the glycan profile in particular with respect to the same antibody having a natural Fc region (the native or wild-type antibody). This has been obtained without altering the MAb cellular reactivity or affinity for DR5.. In accordance with the invention, the Fc mutations may be made in all the existing anti-DR5 antibodies in order to improve their apoptosis potency level. An anti-DR5 antibody that has no marked or significant apoptosis potency may benefit from the present invention and get marked or significant apoptosis potency.

An object of the invention is thus a DR5-binding monoclonal antibody comprising an immunoglobulin domain binding specifically to a DR5 receptor and a variant human IgG Fc region, preferably an IgG1 Fc region, wherein the variant IgG Fc region comprises an amino acid substitution at the amino acid positions 326, 333 and/or 396, more preferably 326 and 333, 326 and 396, 333 and 396 or 326, 333 and 396. The anti-DR5 antibody is strongly apoptotic when bound to the DR5 receptor of a cancer cell. It induces stronger apoptosis with respect to the same antibody that has a non-mutated Fc region or with respect to the same antibody that hasn't the same Fc mutations. A particularly preferred object is such an antibody having an Fc region that does not comprise other amino acid mutations that could involve induction or enhancing or significantly enhancing of ADCC and/or CDC and/or could have an impact on the glycan profile. Preferably, the Fc region will have amino acid mutations consisting essentially of, or consisting of, substitutions at 326 and 333, 326 and 396, 333 and 396 or 326, 333 and 396.

Biological assays are used to investigate on apoptosis such as the MAb inhibition (IC 50) on cell proliferation or on the induction of caspase cleavage. Reference may be made to the following Examples and to the common knowledge of the person skilled in the art. Such assays may be used to determine an improved apoptosis induction by the mutated antibody with respect to the native antibody which differs from the mutated one by the fact that the Fc region is native (unmodified).

Another object is a composition or pharmaceutical composition comprising at least one such antibody and a pharmaceutically acceptable carrier, diluent, or excipient.

Another object of the invention is a method for inducing apoptosis of a DR5-expressing cell involved in a disease, such as cancer, autoimmune disease, inflammation or inflammatory condition, infectious disease, especially viral infection or viral disease, neurological disorder, or any other disease wherein cells expressing a DR5 receptor are involved or present. More particularly, the disease is cancer. The method comprises the administration to a mammal, including human, of an effective amount of an antibody or a composition according to the invention. According to a feature, the method does not induce significant ADCC or comprises the administration of such an antibody that does not induce significant ADCC. According to another feature, the method induces no alteration on functional CDC or does not modulate CDC or comprises the administration of such an antibody that does have a similar CDC than the native or wild-type antibody. According to another feature, the amino acid substitutions in the Fc region of the antibody do not alter DR5 binding affinity and/or do not change the glycan profile.

Another object of the invention is a method for treating a cancer having cancer cells expressing a DR5 receptor. The method comprises the administration to a mammal, including human, of an effective amount of an antibody or a composition according to the invention.

Another object of the invention is a method for treating an autoimmune disease, an inflammatory disease or condition, an infectious disease such as a viral infection or viral disease, a neurological disorder, or any other disease wherein cells expressing a DR5 receptor are involved or present. The method comprises the administration to a mammal, including human, of an effective amount of an antibody or a composition according to the invention.

Another object of the invention is to provide for a method for treating such a disease on a mammal, including human, which has been diagnosed as having DR5-expressing cells involved in the disease or present. Especially, the patient is first diagnosed, then treated with the antibody or composition according to the invention.

These methods lead to apoptosis induction or enhancement (up regulation) of the DR5-expressing targeted cells. The method uses in particular the apoptosis potency of the antibody. According to a feature, the method does not induce significant ADCC or comprises the administration of such an antibody that does not induce significant ADCC. According to another feature, the method induces no alteration on functional CDC or comprises the administration of such an antibody that does have a similar CDC than the native or wild-type antibody.

In a specific embodiment, these methods use an antibody that has enhanced ADCC and/or modulate CDC. In this embodiment, the antibody may have a different glycan profile.

### DEFINITIONS

The terms "apoptosis" and "apoptotic activity" are used in a broad sense and refer to the orderly or controlled form of cell death in mammals that is typically accompanied by one or more characteristic cell changes, including condensation of cytoplasm, loss of plasma membrane microvilli, segmentation of the nucleus, degradation of chromosomal DNA or loss of mitochondrial function. This activity can be determined and measured, for instance, by cell viability assays, FACS analysis or DNA electrophoresis, and more specifically by binding of annexin V, fragmentation of DNA, cell shrinkage, dilation of endoplasmic reticulum, cell fragmentation, and/or formation of membrane vesicles (called apoptotic bodies).

The term "agonist"' and "agonistic" when used herein refer to or describe a molecule which is capable of, directly or indirectly, substantially inducing promoting or enhancing DR5-mediated apoptosis, especially which results in an activation of one more intracellular apoptotic signaling pathway which may include activation of caspase 3, caspase 8, caspase 10 or FADD.

The term "monoclonal antibody" as used herein refers to an antibody obtained from a population of substantially homogeneous antibodies, i.e., the individual antibodies comprising the population are identical except for possible naturally occurring mutations or alternative post-translational modifications that may be present in minor amounts, whether produced from hydridomas or recombinant DNA techniques.

Antibodies are proteins, which exhibit binding specificity to a specific antigen. Native antibodies are usually heterotetrameric glycoproteins of about 150,000 Daltons, composed of two identical light (L) chains and two identical heavy (H) chains. Each light chain is linked to a heavy chain by one covalent disulfide bond, while the number of disulfide linkages varies between the heavy chains of different immunoglobulin isotypes. Each heavy and light chain also has regularly intrachain disulfide bridges.

Each heavy chain has at one end a variable domain (V_{H}) followed by a number of constant domains. Each light chain has a variable domain at one end (V_{L}) and a constant domain at its other end. The constant domain of the light chain is aligned with the first constant domain of the heavy chain, and the light chain variable domain is aligned with the variable domain of the heavy chain.

The term 'variable' refers to the fact that certain portions of the variable domains differ extensively in sequence among antibodies and are responsible for the binding specificity of each particular antibody for its particular antigen. However, the variability is not evenly distributed through the variable domains of antibodies. It is concentrated in three segments called complementarily determining regions (CDRs) both in the light chain and the heavy chain variable domains.

The more highly conserved portions of the variable domains are called the framework regions (FR). The variable domains of native heavy and light chains each comprise four FR regions, largely adopting a β-sheet configuration, connected by three CDRs, which form loops connecting, and in some cases forming part of the β-sheet structure. The CDRs in each chain are held together in close proximity by the FR regions and, with the CDRs from the other chain, contribute to the formation of the antigen binding site of antibodies (Kabat *et al.,* 1991).

The constant domains are not involved directly in binding an antibody to an antigen, but exhibit various effector functions. Depending on the amino acid sequence of the constant region of their heavy chains, antibodies or immunoglobulins can be assigned to different classes. There are five major classes of immunoglobulins: IgA, IgG, IgD, IgE and IgM, and several of these may be further divided into subclasses (isotypes), e.g. IgG1, IgG2, IgG3 and IgG4; IgA1 and IgA2. The heavy chain constant regions that correspond to the different classes of immunoglobulins are called α, δ, ε, γ, and µ, respectively. Of the various human immunoglobulin classes, only IgG1, IgG2, IgG3 and IgM are known to activate complement.

With respect to antibodies of the invention, the term "immunologically specific" or "specifically binds" or "binding specifically to" and similar refers to antibodies that bind to one or more epitopes of a protein of interest (e.g., DR5/TRAIL R2), but which do not substantially recognize and bind other molecules in a sample containing a mixed population of antigenic biological molecules.

"Humanized" forms of non-human (e.g., murine) antibodies are chimeric immunoglobulins which contain minimal sequence derived from non-human immunoglobulin. For the most part, humanized antibodies are human immunoglobulins (recipient antibody) in which residues from a complementarity-determining region (CDR) of the recipient are replaced by residues from a CDR of a non-human species (donor antibody) such as mouse, rat or rabbit having the desired specificity, affinity, and capacity. In some instances, Fv framework region (FR) residues of the human immunoglobulin are replaced by corresponding non-human residues.

In a preferred embodiment, a murine CDR is grafted into the framework region of a human antibody to prepare the "humanized antibody". See, e.g., Riechmann, L. et al., Nature. 1988; 332: 323-327*;* and Neuberger, MS et al., Nature. 1985; 314: 268-270*.*

Furthermore, humanized antibodies may comprise residues which are found neither in the recipient antibody nor in the imported CDR or framework sequences. These modifications are made to further refine and maximize antibody performance. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin sequence. For further details, see Jones et al., Nature. 1986; 321:522-525*;* Reichmann et al., Nature. 1988; 332:323-329*; and* Presta et al., Curr. Op. Struct. Biol. 1992; 2:593-596*.*

Embodiments of the antibodies and compositions are defined by using the CDRs definitions according to IMGT®. However, the invention encompasses and relates also to the equivalent or alternative compositions wherein the IMGT® numbering is replaced either by the Kabat® numbering or the Common numbering system, using the sequences indicated supra. Therefore, in the following embodiments of a composition, other embodiments are part of the invention in which one replaces the CDRs defined with IMGT® numbering, by the Kabat® numbering, in particular in accordance with Tables 1 and 2. Also, in the following embodiments of a composition, other embodiments are part of the invention in which one replaces the CDRs defined with IMGT® numbering, by the Common numbering system, in accordance with the table supra.

"Treatment" or "therapy" refer to both therapeutic treatment and prophylactic or preventative measures.

"Mammal" for purposes of treatment or therapy refers to any animal classified as a mammal, including humans, domestic and farm animals, and zoo, sports, or pet animals, such as dogs, horses, cats, cows, etc. Preferably, the mammal is human.

The terms "cancer" and "cancerous" refer to or describe the physiological condition in mammals that is typically characterized by unregulated cell growth. Examples of cancer include but are not limited to, carcinoma, lymphoma, blastoma, sarcoma, and leukemia. More particular examples of such cancers include squamous cell cancer, small-cell lung cancer, non-small cell lung cancer, gastrointestinal cancer, renal cancer, pancreatic cancer, glioblastoma, cervical cancer, ovarian cancer, liver cancer, bladder cancer, hepatoma, breast cancer, colon cancer, colorectal cancer, endometrial carcinoma, salivary gland carcinoma, kidney cancer, prostate cancer, vulval cancer, thyroid cancer, hepatic carcinoma and various types of head and neck cancer.

The term "nucleic acid" or "oligonucleotide" or grammatical equivalents herein refer to at least two nucleotides covalent linked together. A nucleic acid of the present invention is preferably single-stranded or double stranded and will generally contain phosphodiester bonds.

"Wild type" antibody refers to antibodies with the original Fc sequence such as those described in Table 7. "Native" antibody refers herein to an antibody having an intact natural Fc region called herein Mut0 in accordance with the invention, and the other regions have not been modified.

Amino acid sequence "variants" (or mutants) of the antibody are prepared by introducing appropriate nucleotide changes into the antibody DNA, or by nucleotide synthesis. The modifications do not alter the above mentioned antibody characteristics such as the IgG isotype and antigen binding. These changes primarily concern the amino acid changes in the Fc region as disclosed herein. Other changes may be made which does not change the overall features or properties of the antibodies, for example to improve the yield of the recombinant production, the protein stability and/or to facilitate the purification. Also, as mentioned above, additional changes may be made in the Fc region, as soon as they do not denaturize the intended properties of the antibody, in particular with respect to apoptosis and/or effector functions, as disclosed herein.

By "effective amount" or "sufficient amount" is meant an amount sufficient to achieve a concentration of agonist which is capable of binding to DR5 receptors and inducing the therapeutic effect, in particular activating the apoptosis pathway of DR5⁺ cells involved in the disease, especially transformed cells such as those found in some cancers or infected cells, so as to treat the disease. Such concentrations can be routinely determined by those of skilled in the art. The amount of the compound actually administered will typically be determined by a physician, in the light of the relevant circumstances, including the condition to be treated, the chosen route of administration, the actual compound administered, the age, weight, and response of the individual patient, the severity of the patient's symptoms, etc. It will also be appreciated by those of stalled in the art that the dosage may be dependent on the stability of the administered peptide.

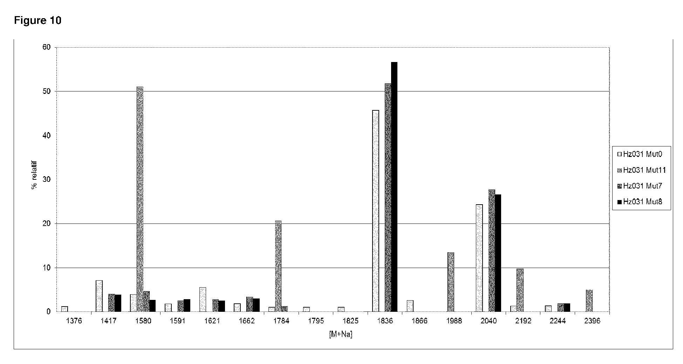

"No glycan profile change" means that the Fc mutations do not dramatically change the glycan profile of the natural Fc region on the detection molecular mass of permethylated glycans, detected as [M+Na]+ by MALDI mass spectrometry. On the contrary, dramatic glycan changes are disclosed for example the appearance or the disappearance of molecular mass of permethylated glycans as shown in Figure 10.

### DETAILED DESCRIPTION OF THE INVENTION

An object of the invention is thus an anti-DR5 monoclonal antibody or a DR5-binding monoclonal antibody comprising an immunoglobulin domain binding specifically to a DR5 receptor and a variant human IgG Fc region, preferably an IgG1 Fc region, wherein the variant IgG Fc region comprises an amino acid substitution at the amino acid positions 326 and 333, 326 and 396, 333 and 396 or 326, 333 and 396.

Preferably, the mutated amino acids at those positions are K326, E333 and P396. More precisely, the substitutions at these positions are K326A, E333A and/or P396L. Still more preferably, the variant human IgG Fc region, preferably IgG1, has substitutions K326A, E333A and P396L. Based on its general knowledge on the amino acids, the person skilled in the art is able to determine other amino acids that may be used to substitute K326, E333 and/or P396 by trial-and-assay routine experimentation

Preferably, typical antibodies of the invention may have a variant IgG Fc region, preferably IgG1 Fc region, comprising amino acid substitutions at those positions : K326 and E333 ; K326 and P396 ; E333 and P396 ; K326, E333 and P396 (List 1), more precisely: K326A and E333A ; K326A and P396L ; E333A and P396L ; K326A, E333A and P396L (List 2). The mutations in the Fc region may comprise, consist essentially of, or consist of mutations of these groups. These antibodies do not induce ADCC and/or do not regulate CDC and/or have no altered glycan profile.

Alternatively, typical antibodies of the invention may have a variant IgG Fc region, preferably IgG1 Fc region, comprising amino acid substitutions at those positions : Y300, K326, E333 and P396 ; F243, R292, Y300, K326, E333 and P396 ; R292, Y300, V305, K326, E333 and P396 ; or F243, R292, Y300, V305, K326, E333 and P396 (List 3), more precisely: Y300L K326A, E333A and P396L ; F243L, R292P, Y300L, K326A, E333A and P396L ; R292P, Y300L, V305L, K326A, E333A and P396L ; or F243L, R292P, Y300L, V305L, K326A, E333A and P396L (List 4). The mutations in the Fc region may comprise, consist essentially of, or consist of mutations of these groups. The additional mutations with respect to 326, 333 and/or 396 are such that they alter, i.e. induce or increase, ADCC and/or alter the glycan profile.

A specific embodiment is an antibody having a variant IgG Fc region, preferably IgG1 Fc region, comprising substitutions K326, E333 and P396 or which comprises solely substitutions at K326, E333 and P396.

In a specific embodiment, the Fc region has mutations consisting of a combination of mutations as disclosed in List 1 and List 2. A particular embodiment is a Fc region having mutations consisting of K326A, E333A and P396L.

In another specific embodiment, the Fc region has mutations "consisting essentially" of one of these combinations of mutations according to List 1 or 2, especially K326A, E333A and P396L. In this context, "essentially" means that the Fc region does not comprise an additional mutation (deletion, substitution or addition) or additional mutations (deletion, substitution and/or addition) that may significantly change the apoptosis potency and effector functions ADCC and CDC and/or the glycan profile of the same antibody that does not have such additional mutation(s).

With respect to an antibody having an Fc region having mutations consisting essentially of K326A, E333A and P396L, "consisting essentially" means that the Fc region does not comprise an additional mutation (deletion, substitution or addition) or additional mutations (deletion, substitution and/or addition) that may significantly change the apoptosis potency and increase effector functions ADCC and CDC and/or the glycan profile of the same antibody that does not have such additional mutation(s).

The antibody of the invention may comprise the immunoglobulin domain binding to DR5 of any existing anti-DR5 antibody or the ones more specifically defined herein with respect to Tables 1 and 2 and in WO2014009358 which is hereby incorporated by reference and to which the person skilled in the art may refer. In some embodiments, the antibody is Conatumumab, Drozitumumab, Lexatumumab, Tigatuzumab or LYb5 wherein the Fc region is as disclosed herein in Table 7. In some embodiments, the antibody comprises an Fc region as disclosed herein and the VH and VL regions as disclosed in IMGT INN 9029, IMGT INN 9255, IMGT INN 8753, IMGT INN 8979 or WO2008066854 (H-Gamma and L-Lambda or Kappa chains) or those disclosed in WO2014009358 (SEQ ID NO: 2 or 6 (VH) and 4 or 8 (VL) as disclosed in this application). The VH and VL sequences disclosed in these documents are incorporated herein by reference and the person skilled in the art may refer to these references.

The CDR sequences may be defined herein in accordance with IMGT®, Kabat® or the Common numbering system which retain the common sequence between IMGT® and Kabat®.

In an embodiment, in addition to a variant Fc as described above, the antibody is an antibody comprising the CDRs appearing on Table 1, preferably a humanized antibody Hz033 with the murine CDRs appearing on Table 1 and human Framework Regions (FR). A specific embodiment is such an antibody having a variant IgG, preferably IgG1 Fc region, comprising substitutions K326, E333 and P396 or which comprises solely substitutions at K326, E333 and P396, e.g. consisting essentially of K326A, E333A and P396L substitutions.

| Table 1 | SEQ ID NO: | Sequence IMGT® | SEQ ID NO: | Sequence Kabat® | SEQ ID NO: | Sequence (Common numbering system) |
|---|---|---|---|---|---|---|
| VH Hz033 | | | | | | |
| CDR1 | 13 | GFNIKDTF | 22 | DTFIH | 32 | KDTF |
| CDR2 | 14 | IDPANGNT | 23 | RIDPANGNTKYDPKFQG | 14 | IDPANGNT |
| CDR3 | 15 | VRGLYTYYFDY | 24 | GLYTYYFDY | 24 | GLYTYYFDY |

| VL Hz033 | | | | | | |
|---|---|---|---|---|---|---|
| CDR1 | 16 | QSISNN | 25 | RASQSISNNLH | 16 | QSISNN |
| CDR2 | | FAS | 26 | FASQSIT | | FAS |
| CDR3 | 17 | QQGNSWPYT | 17 | QQGNSWPYT | 17 | QQGNSWPYT |

In another embodiment, in addition to a variant Fc as described above, the antibody is an antibody comprising the CDRs appearing on Table 2, preferably a humanized antibody Hz031 with the murine CDRs appearing on Table 2 and human FR. A specific embodiment is such an antibody having a variant IgG, preferably IgG1 Fc region, comprising substitutions K326, E333 and P396 or which comprises solely substitutions at K326, E333 and P396, e.g. consisting essentially of K326A, E333A and P396L substitutions.

| Table 2 | SEQ ID NO: | Sequence IMGT® | SEQ ID NO: | Sequence Kabat® | SEQ ID NO: | Sequence (Common numbering system) |
|---|---|---|---|---|---|---|
| VH Hz031 | | | | | | |
| CDR1 | 18 | GFNIKDTH | 27 | DTHMH | 33 | KDTH |
| CDR2 | 14 | IDPANGNT | 28 | RIDPANGNTEYDQKFQG | 14 | IDPANGNT |
| CDR3 | 19 | ARWGTNVYFAY | 29 | WGTNVYFAY | 29 | WGTNVYFAY |

| VL Hz031 | | | | | | |
|---|---|---|---|---|---|---|
| CDR1 | 20 | SSVSY | 30 | SASSSVSYMY | 20 | SSVSY |
| CDR2 | | RTS | 31 | RTSNLAS | | RTS |
| CDR3 | 21 | QQYHSYPPT | 21 | QQYHSYPPT | 21 | QQYHSYPPT |

By definition, these CDRs include variant CDRs, by deletion, substitution or addition of one or more amino acid(s), which variant keeps the specificity of the original CDR. The common numbering system provides for a CDR definition having the shortest amino acid sequences or the minimal CDR definition.

Hz031 and Hz033 may have the VH and VL amino acid sequences and nucleic acid sequences are depicted on the following tables:

| Table 3 | Amino acid sequence VH | Amino acid sequence VL |
|---|---|---|
| Hz033 | SEQ ID NO: 2 | SEQ ID NO: 4 |
| Hz031 | SEQ ID NO: 6 | SEQ ID NO: 8 |

| Table 4 | Nucleic acid sequence VH | Nucleic acid sequence VL |
|---|---|---|
| Hz033 | SEQ ID NO: 1 | SEQ ID NO: 3 |
| Hz031 | SEQ ID NO: 5 | SEQ ID NO: 7 |

Hz031 and Hz033 have the CH and CL amino acid sequences and nucleic acid sequences are depicted on the following Table 5

| Table 5 | CH | CL |
|---|---|---|
| Amino acid sequence | SEQ ID NO: 10 in which the selected mutations according to the invention are made | SEQ ID NO: 12 |
| Nucleic acid sequence | SEQ ID NO: 9 in which the selected mutations according to the invention are made | SEQ ID NO: 11 |

Embodiments will now be described of monoclonal antibodies comprising an Fc region according to the invention, and a variable region defined by their CDRs. The FRs are preferably of human origin. Preferably, the IgG, typically IgG1 Fc region, comprises substitutions K326, E333 and P396 or comprises solely substitutions at K326, E333 and P396, e.g. consisting essentially of K326A, E333A and P396L substitutions.

In an embodiment, the antibody comprises one or two binding domains comprising a pair of VH and VL chains wherein the VH chain contains a CDR1 of sequence SEQ ID NO: 13, a CDR2 of sequence SEQ ID NO: 14, a CDR3 of sequence SEQ ID NO: 15; and the VL chain contains a CDR1 of sequence SEQ ID NO: 16, a CDR2 of sequence FAS, a CDR3 of sequence SEQ ID NO: 17. This polypeptide binds specifically to a first epitope on the DR5 receptor.

In an embodiment, the antibody comprises one or two binding domains comprising a pair of VH and VL chains wherein the VH chain contains a CDR1 of sequence SEQ ID NO: 22, a CDR2 of sequence SEQ ID NO: 23, a CDR3 of sequence SEQ ID NO: 24; and the VL chain contains a CDR1 of sequence SEQ ID NO: 25, a CDR2 of sequence SEQ ID NO: 26, a CDR3 of sequence SEQ ID NO: 17. This polypeptide binds specifically to the same first epitope on the DR5 receptor.

In an embodiment, the antibody comprises one or two binding domains comprising a pair of VH and VL chains wherein the VH chain contains a CDR1 of sequence SEQ ID NO : 32, a CDR2 of sequence SEQ ID NO: 14, a CDR3 of sequence SEQ ID NO: 24; and the VL chain contains a CDR1 of sequence SEQ ID NO: 16, a CDR2 of sequence FAS, a CDR3 of sequence SEQ ID NO: 17. This polypeptide binds specifically to the same first epitope on the DR5 receptor.

In an embodiment, the antibody comprises a pair of VH and VL chains wherein the VH chain contains a CDR1 of sequence SEQ ID NO: 18, a CDR2 of sequence SEQ ID NO: 14, a CDR3 of sequence SEQ ID NO: 19; and the VL chain contains a CDR1 of sequence SEQ ID NO: 20, a CDR2 of sequence RTS, a CDR3 of sequence SEQ ID NO: 21. This polypeptide binds specifically to a second and different epitope on the DR5 receptor.

In an embodiment, the antibody comprises a pair of VH and VL chains wherein the VH chain contains a CDR1 of sequence SEQ ID NO: 27, a CDR2 of sequence SEQ ID NO: 28, a CDR3 of sequence SEQ ID NO: 29; and the VL chain contains a CDR1 of sequence SEQ ID NO: 30, a CDR2 of sequence SEQ ID NO: 31, a CDR3 of sequence SEQ ID NO: 21. This polypeptide binds specifically to the same second epitope on the DR5 receptor.

In an embodiment, the antibody comprises a pair of VH and VL chains wherein the VH chain contains a CDR1 of sequence SEQ ID NO: 33, a CDR2 of sequence SEQ ID NO: 14, a CDR3 of sequence SEQ ID NO: 29; and the VL chain contains a CDR1 of sequence SEQ ID NO: 20, a CDR2 of sequence RTS, a CDR3 of sequence SEQ ID NO: 21. This polypeptide binds specifically to the same second epitope on the DR5 receptor.

In an embodiment, the antibodies as defined herein comprise the same two binding domains.

In another embodiment, the antibody comprises two binding domains and these two binding domains are each specific of a different epitope on the DR5 receptor. These binding domains may comprise a specific set of 3 CDRs on the VH and VL as disclosed and provided therein and may be identical or slightly different in the framework regions.

In an embodiment, the antibody is bispecific or biparatopic and comprises:
- a first binding domain comprising a pair of VH and VL chains wherein the VH chain contains a CDR1 comprising or consisting of sequence SEQ ID NO: 13, a CDR2 comprising or consisting of sequence SEQ ID NO: 14, a CDR3 comprising or consisting of sequence SEQ ID NO: 15; and the VL chain contains a CDR1 comprising or consisting of sequence SEQ ID NO: 16, a CDR2 comprising or consisting of sequence FAS, a CDR3 comprising or consisting of sequence SEQ ID NO: 17, and
- a second binding domain comprising a pair of VH and VL chains wherein the VH chain contains a CDR1 comprising or consisting of sequence SEQ ID NO: 18, a CDR2 comprising or consisting of sequence SEQ ID NO: 14, a CDR3 comprising or consisting of sequence SEQ ID NO: 19; and the VL chain contains a CDR1 comprising or consisting of sequence SEQ ID NO: 20, a CDR2 comprising or consisting of sequence RTS, a CDR3 comprising or consisting of sequence SEQ ID NO: 21.

In an embodiment, the antibody is bispecific or biparatopic and comprises:
- a first binding domain comprising a pair of VH and VL chains wherein the VH chain contains a CDR1 comprising or consisting of sequence SEQ ID NO:22, a CDR2 comprising or consisting of sequence SEQ ID NO: 23, a CDR3 comprising or consisting of sequence SEQ ID NO: 24; and the VL chain contains a CDR1 comprising or consisting of sequence SEQ ID NO: 25, a CDR2 comprising or consisting of sequence SEQ ID NO: 26, a CDR3 comprising or consisting of sequence SEQ ID NO: 17, and
- a second binding domain comprising a pair of VH and VL chains wherein the VH chain contains a CDR1 comprising or consisting of sequence SEQ ID NO: 27, a CDR2 comprising or consisting of sequence SEQ ID NO: 28, a CDR3 comprising or consisting of sequence SEQ ID NO: 29; and the VL chain contains a CDR1 comprising or consisting of sequence SEQ ID NO: 30, a CDR2 comprising or consisting of sequence SEQ ID NO: 31, a CDR3 comprising or consisting of sequence SEQ ID NO: 21.

In an embodiment, the antibody is bispecific or biparatopic and comprises:
- a first binding domain comprising a pair of VH and VL chains wherein the VH chain contains a CDR1 comprising or consisting of sequence SEQ ID NO: 32, a CDR2 comprising or consisting of sequence SEQ ID NO: 14, a CDR3 comprising or consisting of sequence SEQ ID NO: 24; and the VL chain contains a CDR1 comprising or consisting of sequence SEQ ID NO: 16, a CDR2 comprising or consisting of sequence FAS, a CDR3 comprising or consisting of sequence SEQ ID NO: 17, and
- a second binding domain comprising a pair of VH and VL chains wherein the VH chain contains a CDR1 comprising or consisting of sequence SEQ ID NO: 33, a CDR2 comprising or consisting of sequence SEQ ID NO: 14, a CDR3 comprising or consisting of sequence SEQ ID NO: 29; and the VL chain contains a CDR1 comprising or consisting of sequence SEQ ID NO: 20, a CDR2 comprising or consisting of sequence RTS, a CDR3 comprising or consisting of sequence SEQ ID NO: 21.

In some embodiments, the antibody of the invention comprises:
- one or more of amino acid sequence pairs SEQ ID NO : 2 and 4 (VH and VL from Hz033) and SEQ ID NO: 6 and 8 (VH and VL from Hz031),
- the pair of amino acid sequences SEQ ID NO : 2 and 4, (VH and VL from Hz033)
- the pair of amino acid sequences SEQ ID NO : 6 and 8, (VH and VL from Hz031) or
- both amino acid sequence pairs SEQ ID NO : 2 and 4 and SEQ ID NO: 6 and 8.

In some embodiments, the antibody of the invention comprises:
- two of each amino acid sequences SEQ ID NO: 2, 10, 4 and 12 (e.g. the whole or intact Hz033 antibody)
- amino acid sequences SEQ ID NO: 2, 10, 4 and 12 (single chain Fv based on Hz033);
- two of each amino acid sequences SEQ ID NO: 6, 10, 8 and 12 (e.g. the whole or intact Hz031 antibody)
- amino acid sequences SEQ ID NO: 6, 10, 8 and 12 (single chain Fv based on Hz031);
- amino acid sequences SEQ ID NO: 2, 6, 4, 8, 10 and 12 (bispecific antibody), especially the bispecific antibody comprises SEQ ID NO: 2, 6, 4, 8, (one of each) and 10, 12 (two of each);
- amino acid sequences SEQ ID NO: 4 and 12 (light chain);
- amino acid sequences SEQ ID NO: 8 and 12 (light chain);
- amino acid sequences SEQ ID NO: 2 and 10 (heavy chain);
- amino acid sequences SEQ ID NO: 6 and 10 (heavy chain),
   wherein SEQ ID NO: 10 has the amino acid substitutions in the Fc region according to the invention, preferably substitutions K326, E333 and P396 or which comprises solely substitutions at K326, E333 and P396.

The antibodies of the invention may be fully murine, say they comprise amino acid sequences that match with the amino acid sequence of the maternal or original murine antibody. The polypeptides of the invention may also be chimeric or humanized, say they can comprise human-derived amino acid sequences. Specifically, the polypeptide may comprise framework regions and/or constant regions of a human-derived antibody.

Another object of the invention is a composition or pharmaceutical composition comprising one, two or more anti-DR5 antibodies according to the invention, as disclosed above and provided herein, and a pharmaceutically acceptable carrier, diluent or excipient.

The present invention also relates to these compositions comprising at least two antibodies according to the invention, for a simultaneous, separate or sequential administration to a mammal, including man.

In an embodiment, the composition comprises two anti-DR5 antibodies, one having the CDRs sequences as defined above with respect to Table 1, the other having the CDRs sequences as defined above with respect to Table 2.

The compositions may further comprise at least one additional antibody directed against another target, for a simultaneous, separate or sequential administration with antibody(ies) of the invention, to a mammal, including man.

The composition comprising two different antibodies may be under the form of a kit comprising both antibodies, in separate conditioning, e.g. in different vials.

Another object of the invention is an antibody or composition according to the invention, as a medicament, more particularly an anti-cancerous medicament, a medicament against autoimmune disease, a medicament against inflammation or inflammatory condition, a medicament against infectious disease, e.g. against viral infection or against viral disease, a medicament against neurological disorder. More specifically, the antibody or composition according to the invention is for use as a medicament to induce DR5-expressing cells apoptosis, where the DR5-expressing cells are involved in the disease. Such cells have been described *supra.* Another object of the invention is the use of an antibody or composition according to the invention, for the manufacture of a medicament against cancer, autoimmune disease, inflammatory disease or condition, infectious disease, e.g. viral infection or disease, or neurological disorder, in particular which induces DR5-expressing cell apoptosis. Such cells have been described *supra.* In an embodiment, the antibody has a variant IgG Fc region comprising amino acid substitutions at the amino acid positions 326 and 333 ; 326 and 396 ; 333 and 396; or 326, 333 and 396, and does not induce ADCC and/or does not regulate CDC and/or no altered glycan profile. In this embodiment, the antibody may, as explained before, comprise additional mutations that do not change this dramatically. In another embodiment, the antibody has a variant IgG Fc region comprising amino acid substitutions at the amino acid positions 326 and 333 ; 326 and 396 ; 333 and 396; or 326, 333 and 396, and has ADCC or increased ADCC and/or an altered glycan profile, due to at least one addition mutation, such as those mentioned herein.

The present invention also relates to a method of prevention and/or treatment of a disease wherein inducing apoptosis of some cells is beneficial to the mammal, in particular the human in terms of prevention or treatment (therapeutic or prophylactic). These diseases are those in which DR5-expressing cells are implicated and for which it is beneficial to the patient by inducing the death of these cells, e.g. to prevent or cure the disease, to decrease the symptoms or to increase the survival time. Those diseases are in particular cancer, especially one of those listed in the Definitions supra, autoimmune disease, inflammatory disease or condition, infectious disease, e.g. viral infection and viral disease, neurological disorder. This method comprises the administration to a mammal, including human, of an effective amount of a composition as disclosed and provided herein. The method comprises the administration at least one optimized anti-DR5 antibody according to the invention. In an embodiment, the antibodies do not induce ADCC, no modulation of functional CDC and/or no glycan profile impact.

This method may comprise administering an effective amount of any anti-DR5 antibody having a mutated Fc region as described. As mentioned earlier, a preferred embodiment is for an antibody having an Fc comprising substitutions at K326, E333 and P396 or which comprises substitutions consisting of or consisting essentially of substitutions at K326, E333 and P396, especially with K326A, E333A and/or P396L. In addition, as explained earlier, the antibody comprises an immunoglobulin domain binding to DR5, wherein this binding domain may be one of any existing anti-DR5 antibody, especially as defined in IMGT INN 9029, IMGT INN 9255, IMGT INN 8753 or IMGT INN 8979 or the ones more specifically defined herein with respect to Tables 1 and 2 and in WO 2014009358. In an embodiment, the antibody does not induce ADCC and/or does not regulate CDC and/or have no altered glycan profile, as disclosed herein. In another embodiment the antibody has ADCC and/or an altered glycan profile due to Fc mutation, as disclosed herein.

Pharmaceutical compositions, uses and methods of treatment are thus intended for the prevention and/or treatment of cancer. A list of cancers that may beneficiate from the invention is given supra in the Definitions.

Pharmaceutical compositions, uses and methods of treatment are thus also intended for the prevention and/or treatment of autoimmune diseases and inflammatory conditions.

The pharmaceutical compositions, uses and methods of treatment are thus also intended for the prevention and/or treatment of viral infection or viral diseases. Viral infections and diseases include, but are not limited to, infections with cytomegalovirus, influenza, vesicular stomatitis virus, herpes simplex virus, hepatitis, adenovirus-2, bovine viral diarrhoea virus, human immunodeficiency virus (HIV), and Epstein-Barr virus.

The pharmaceutical compositions can be administered by injection, that is, intravenously, intramuscularly, intracutaneously, subcutaneously, intraduodenally or intraperitoneally. Also in certain embodiments, the compounds can be administered by inhalation, for example, intranasally. Other pharmaceutical delivery systems can also be employed, for example, liposomes.

Mammalian cells are the preferred hosts for production of therapeutic glycoproteins, due to their capability to glycosylate proteins in the most compatible form for human applications. Bacteria very rarely glycosylate proteins, and like other type of common hosts, such as yeasts, filamentous fungi, insect and plant cells yield glycosylation patterns associated with rapid clearance from the blood stream.

Among mammalian cells, Chinese hamster ovary (CHO) cells are the most commonly used. In addition to giving suitable glycosylation patterns, these cells allow consistent generation of genetically stable, highly productive clonal cell lines. They can be cultured to high densities in simple bioreactors using serum-free media, and permit the development of safe and reproducible bioprocesses. Other commonly used animal cells include baby hamster kidney (BHK) cells, NSO- and SP2/0-mouse myeloma cells.

In an embodiment, the monoclonal antibodies according to the invention are produced or expressed in mammal cells, preferably wild-type mammal cells, preferably of rodent origin, especially CHO cells.

Modifications and changes may be made in the structure of an antibody of the present invention and still obtain a molecule having like characteristics. For example, certain amino acids can be substituted for other amino acids in a sequence without appreciable loss of activity. Because it is the interactive capacity and nature of a polypeptide that defines that polypeptide's biological functional activity, certain amino acid sequence substitutions can be made in a polypeptide sequence (or, of course, its underlying DNA coding sequence) and nevertheless obtain a polypeptide with like properties.

In making such changes, the hydropathic index of amino acids can be considered. The importance of the hydropathic amino acid index in conferring interactive biologic function on a polypeptide is generally understood in the art. It is known that certain amino acids can be substituted for other amino acids having a similar hydropathic index or score and still result in a polypeptide with similar biological activity. Each amino acid has been assigned a hydropathic index on the basis of its hydrophobicity and charge characteristics.

It is believed that the relative hydropathic character of the amino acid determines the secondary structure of the resultant polypeptide, which in turn defines the interaction of the polypeptide with other molecules, for example, enzymes, substrates, receptors, antibodies, antigens, and the like. It is known in the art that an amino acid may be substituted by another amino acid having a similar hydropathic index and still obtain a biologically functionally equivalent polypeptide. In such changes, the substitution of amino acids whose hydropathic indices are within ±2 is preferred, those which are within ±1 are particularly preferred, and those within +0.5 are even more particularly preferred.

Substitution of like amino acids can also be made on the basis of hydrophilicity, particularly where the biologically functionally equivalent peptide or polypeptide thereby created is intended for use in immunological embodiments. U.S. Patent 4,554,101, incorporated herein by reference or to which the person skilled in the art : may refer, states that the greatest local average hydrophilicity of a polypeptide, as governed by the hydrophilicity of its adjacent amino acids, correlate with its immunogenicity and antigenicity, *i.e.* with a biological property of the polypeptide.

As detailed in U.S. Patent 4,554,101, the following hydrophilicity values have been assigned to amino acid residues: arginine (+3.0); lysine (+3.0); aspartate (+3.0 ±1); glutamate (+3.0 ±1); serine (+0.3); asparagine (+0.2); glutamine (+0.2); glycine (0); proline (-0.5 ±1); threonine (-0.4); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4). It is understood that an amino acid can be substituted for another having a similar hydrophilicity value and still obtain a biologically equivalent, and in particular, an immunologically equivalent, polypeptide. In such changes, the substitution of amino acids whose hydrophilicity values are within ±2 is preferred, those which are within ±1 are particularly preferred, and those within ±0.5 are even more particularly preferred.

As outlined above, amino acid substitutions are generally therefore based on the relative similarity of the amino acid side-chain substituents, for example, their hydrophobicity, hydrophilicity, charge, size, and the like: Table 5:

| Amino Acid | Index | Amino Acid | Index |
|---|---|---|---|
| isoleucine L | (+4,5) | tryptophan W | (-0,9) |
| valine V | (+4,2) | tyrosine Y | (-1,3) |
| leucine L | (+3,8) | proline P | (-1,6) |
| phenylalanine | (+2,8) | histidine H | (-3,2) |
| cysteine C | (+2,5) | glutamate E | (-3,5) |
| methionine M | (+1,9) | glutamine Q | (-3,5) |
| alanine A | (+1,8) | aspartate D | (-3,5) |
| glycine G | (-0,4) | asparagine N | (-3,5) |
| threonine T | (-0,7) | lysine K | (-3,9) |
| serine S | (-0,8) | arginine R | (-4,5) |

Amino acid substitution may be chosen or selected differently. Possible substitutions have been documented in WO99/51642, WO2007024249 and WO2007106707.

Based on its general knowledge on the amino acids, the person skilled in the art is able, including by trial-and-assay routine experimentation, to determine amino acids and positions that may be deleted, added or substituted without significantly changing the functionality of the Fc region or of any other part of the antibody, including the binding domain.

By definition, the CDRs of the invention include variant CDRs, by deletion, substitution or addition of one or more amino acid(s), which variant keeps the specificity of the original CDR. The common numbering system provides for a CDR definition having the shortest amino acid sequences or the minimal CDR definition.

Methods for producing the antibodies are known from the person skilled in the art. The mammal cells, preferably rodent cells such as CHO cells, preferably wild-type cells are transfected with one or several expression vectors. Preferably, the cells are co-transfected with an expression vector for light chain and with an expression vector for heavy chain.

Cell transfection is also known from the person skilled in the art. As transfection that may be performed, one may mention without limitation standard transfection procedures, well-known from the man skilled in the art, such as calcium phosphate precipitation, DEAE-Dextran mediated transfection, electroporation, magnetofection, nucleofection (AMAXA Gmbh, Lonza), liposome-mediated transfection (using dreamfect®, lipofectin®, lipofectamine® or Hype5 technology for example) or microinjection.

Expression vectors are known. As vectors that may be used, one may mention without limitation: pcDNA3.3, pOptiVEC, pFUSE, pMCMVHE, pMONO, pSPORT1, pcDV1, pcDNA3, pcDNA1, pRc/CMV, pSEC. One may use a single expression vector or several expression vectors expressing different parts of the polypeptide or antibody.

The isolated nucleic acid sequences disclosed and provided herein are also objects of the invention. Thus the invention particularly relates to an isolated nucleotide coding for an antibody Heavy Chain, comprising sequences SEQ ID NO: 1 and 9, or 5 and 9; or an isolated nucleotide sequence coding for an antibody Light Chain comprising sequences SEQ ID NO: 3 and 11, or 7 and 11. The present invention also concerns an expression vector comprising such nucleotide sequence and the regulatory elements allowing expression of the sequences. The invention also concerns the host cells comprising such vector.

The person skilled in the art fully owns the methods to generate the antibodies according to the invention using such a vector or vectors and cells such as CHO cells.

The present invention will now be described in further detail by way of examples referring to the figure. Note that in the block diagrams, the blocks appear from the left to the right in the same order than indicated in the legend in the diagrams where the legend is put in a box.

### BRIEF DESCRIPTION OF THE DRAWINGS

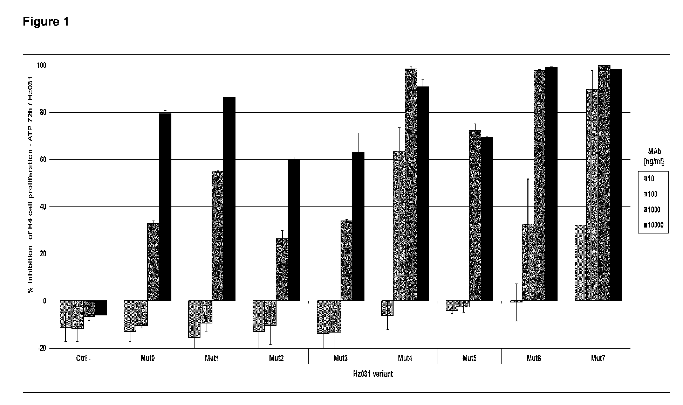

**Figure 1** is a bar diagram showing percent (%) of H4 cell proliferation inhibition (ATP bioluminescent bioassay, 72 hours) of Hz031 related anti-DR5 native (Mut0) antibody alone tested as compared to MAb variant Mut1 (E333A), Mut2 (K326A), Mut3 (P396L) Mut4 (K326A E333A), Mut5 (K326A P396L), Mut6 (E333A P396L) or Mut7 (K326A E333A P396L) tested at different concentrations, (mean +/- SD, n=2).

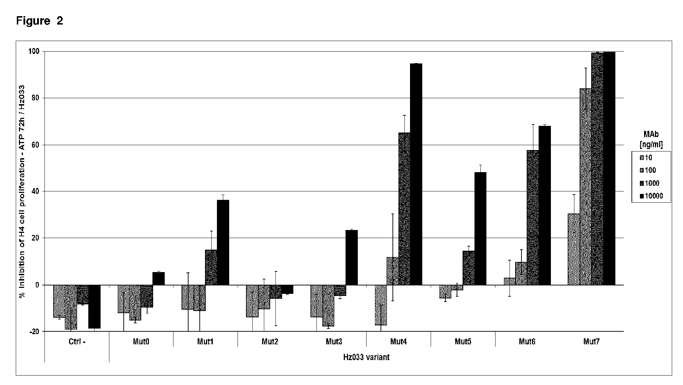

**Figure 2** is a bar diagram showing percent (%) of the H4 cell proliferation inhibition (ATP bioluminescent bioassay, 72 hours) of Hz033 related anti-DR5 native (Mut0) antibody alone tested as compared to MAb variant Mut1 (E333A), Mut2 (K326A), Mut3 (P396L) Mut4 (K326A E333A), Mut5 (K326A P396L), Mut6 (E333A P396L) or Mut7 (K326A E333A P396L) tested at different concentrations, (mean +/- SD, n=2).

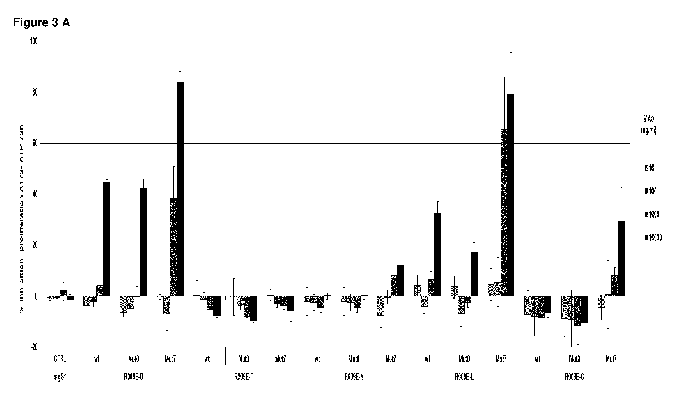

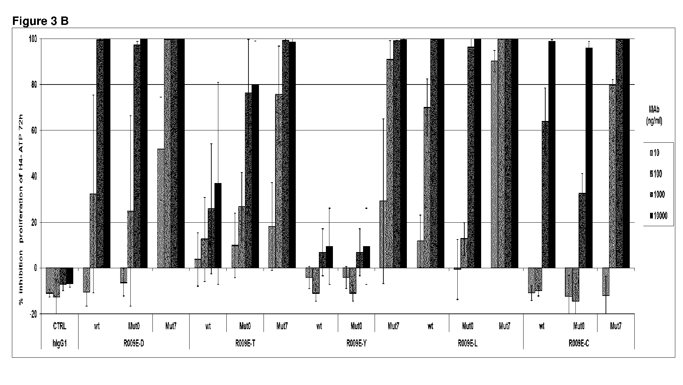

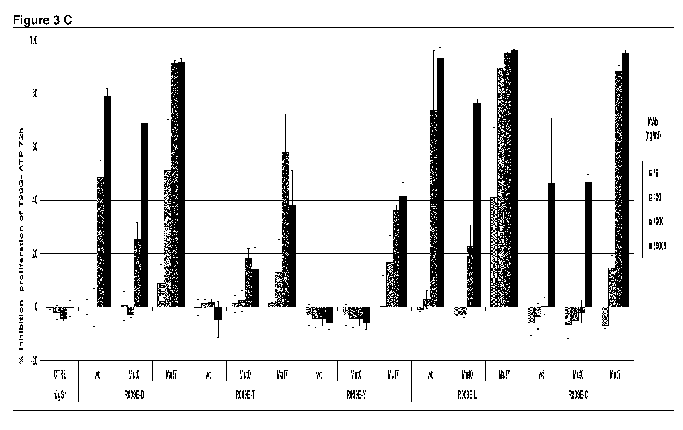

**Figure 3** is a bar diagram showing percent (%) of cell proliferation inhibition (ATP bioluminescent bioassay, 72 hours) on A172 (Figure 3A), on H4 (Figure 3B) and T98G (Figure 3C) of R009E-D, R009E-T, R009E-Y, R009E-L or R009E-C related anti-DR5 wild type (wt), native (Mut0) antibody alone tested as compared to MAb variant Mut7 (K326A E333A P396L) tested at different concentrations (mean +/- SD, n=2)

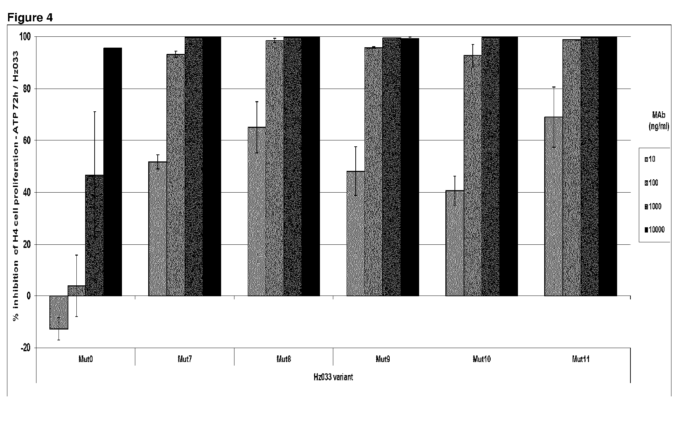

**Figure 4** is a bar diagram showing percent (%) of H4 cell proliferation inhibition (ATP bioluminescent bioassay, 72 hours) of Hz033 related anti-DR5 native antibody alone tested as compared to MAb variant Mut7 (K326A E333A P396L), Mut8 (Y300L K326A E333A P396L), Mut9 (F243L R292P Y300L K326A E333A P396L), Mut10 (R292P Y300L V305L K326A E333A P396L) or Mut11 (F243L R292P Y300L V305L K326A E333A P396L) tested at different concentrations, (mean +/- SD, n=2).

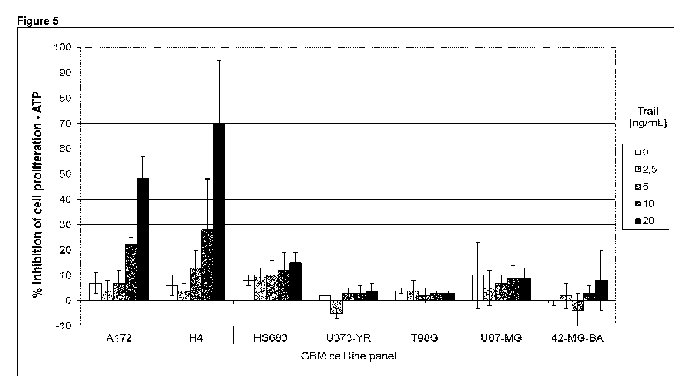

**Figure 5** is a bar diagram showing percent (%) of cell proliferation inhibition (ATP bioluminescent bioassay, 72 hours) in the presence of the recombinant TRAIL tested at different concentrations on A172, H4, HS683, U373, T98G; U87MG and 42MG-BA, (mean +/- SD, n=2).

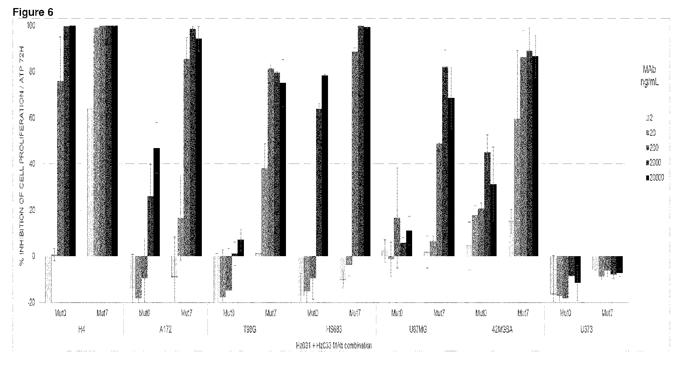

**Figure 6** is a bar diagram showing percent (%) of cell proliferation inhibition (ATP bioluminescent bioassay, 72 hours) of Mut7 (K326A E333A P396L) versus Mut0 anti-DR5 antibody combination (Hz031+Hz033) tested at different concentrations on A172, H4, HS683, U373, T98G; U87MG and 42MG-BA cells, (mean +/- SD, n=2).

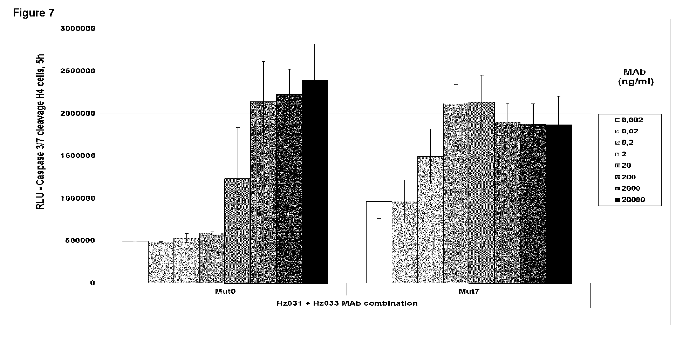

**Figure 7** is a bar diagram showing of the relative luminescence units (RLU) of cleaved caspase 3/7 quantification of Mut7 (K326A E333A P396L) anti-DR5 antibody combination (Hz031+Hz033) versus native (Mut0) antibody combination, tested at different concentrations on H4 cells (mean +/- SD, n=2).

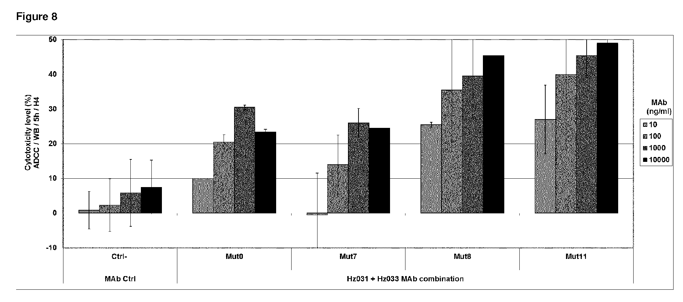

**Figure 8** is a bar diagram showing percent (%) of MAb triggered ADCC in H4 cells of Mut7 (K326A E333A P396L), Mut8 (Y300L K326A E333A P396L), Mut11 (F243L R292P Y300L V305L K326A E333A P396L) versus native (Mut0) anti-DR5 antibody combination (Hz031+Hz033), tested at different concentrations, (mean +/- SD, n=2).

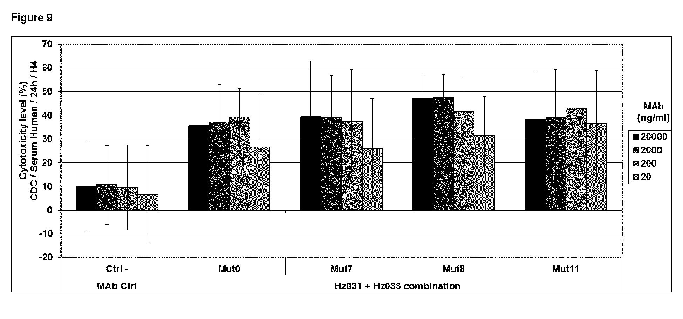

**Figure 9** is a bar diagram showing percent (%) of MAb triggered CDC in H4 cells of Mut7 (K326A E333A P396L), Mut8 (Y300L K326A E333A P396L), Mut11 (F243L R292P Y300L V305L K326A E333A P396L) versus native (Mut0) anti-DR5 antibody combination (Hz031+Hz033), tested at different concentrations, (mean +/- SD, n=2).
**Figure 10****:** N-glycosylation profile analyzed by MALDI-TOF mass spectrum of native (Mut0) compared to Mut7 ((K326A E333A P396L), Mut8 (Y300L K326A E333A P396L) or Mut11 (F243L R292P Y300L V305L K326A E333A P396L) from Hz031 MAb variants produced from wild type CHO cells. Representative experiment *(n=1).* Similar data was obtained from Hz033 MAb variants

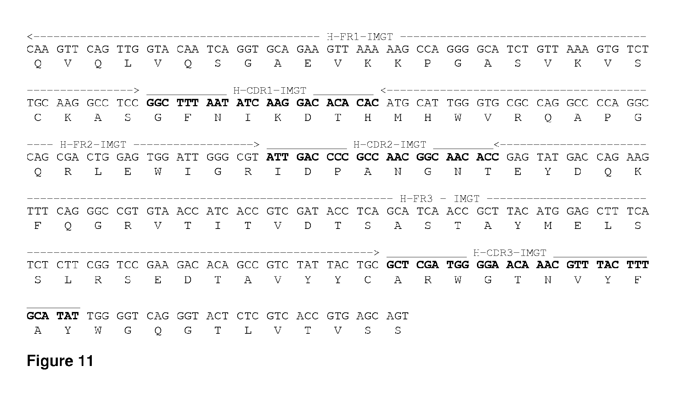

**Figure 11****:** Amino acid and nucleic acid sequence for VH Hz031 with description of the FR1, CDR1, FR2, CDR2, FR3, CDR3 defining according IMGT®.

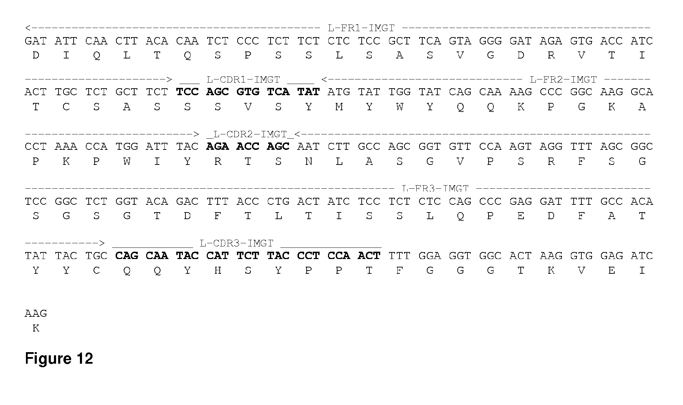

**Figure 12****:** Amino acid and nucleic acid sequence for VL Hz031 with description of the FR1, CDR1, FR2, CDR2, FR3, CDR3 defining according IMGT®.

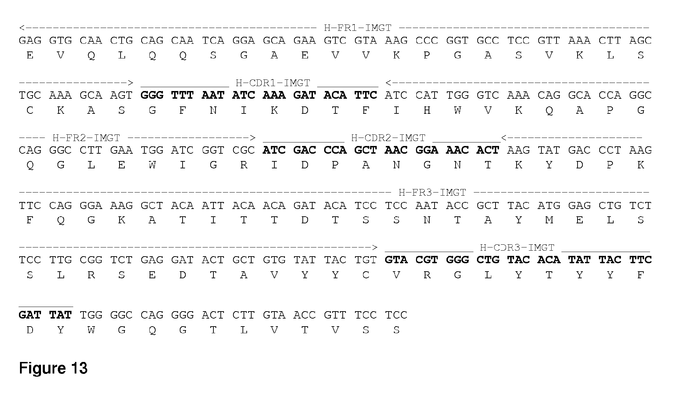

**Figure 13****:** Amino acid and nucleic acid sequence for VH Hz033 with description of the FR1, CDR1, FR2, CDR2, FR3, CDR3 defining according IMGT®.

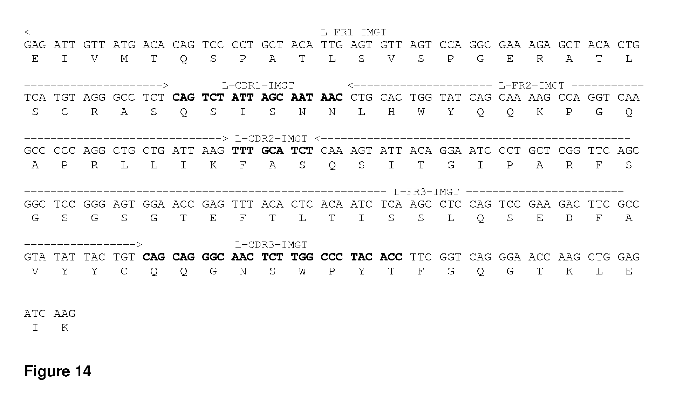

**Figure 14****:** Amino acid and nucleic acid sequence for VL Hz033 with description of the FR1, CDR1, FR2, CDR2, FR3, CDR3 defining according IMGT®.

### EXAMPLES

The following examples are offered to illustrate, but not to limit the claimed invention.

### MATERIALS AND METHODS

### Cell culture

The established human neuroglioma cells H4, HS683, A172 and U373 (available from ATCC) were grown in Dulbecco's Modified Eagle's Medium (Sigma, St Quentin Fallavier, France) supplemented with 10% heat-inactivated fetal bovine serum (FBS), (Sigma, St Quentin Fallavier, France), 4 nM L-glutamine (Sigma, St Quentin Fallavier, France) and 100 U/mL, 100 µg/mL penicillin - streptomycine (Sigma, St Quentin Fallavier, France).

The established human glioblastoma astrocytoma cells U87MG or T98G were grown in Eagle's Minimum Essential Medium (Sigma, St Quentin Fallavier, France) supplemented with 10% heat-inactivated fetal bovine serum (FBS) (Sigma, St Quentin Fallavier, France), 4 nM L-glutamine (Sigma, St Quentin Fallavier, France) and 100 U/mL, 100 µg/mL penicillin - streptomycine (Sigma, St Quentin Fallavier, France).

The established human glioma cells 42MGBA (available from DSMZ) were grown in 80% mixture of RPMI-1640 Medium and Eagle's Minimum Essential Medium at 1:1 (Sigma, St Quentin Fallavier, France) supplemented with 20% heat-inactivated fetal bovine serum (FBS) (Sigma, St Quentin Fallavier, France), 4 nM L-glutamine (Sigma, St Quentin Fallavier, France) and 100 U/mL, 100 µg/mL penicillin - streptomycine (Sigma, St Quentin Fallavier, France).

### Construction of antibody variants

Substitutions in the Fc domain were introduced using "megaprimer" method of site-directed mutagenesis (Sarkar et al., Biotechniques Vol 8, N°4 (1990), 404-407). Positions are numbered according to the Kabat® index (Identical V region amino acid sequences and segments of sequences in antibodies of different specificities). Relative contributions of VH and VL genes, minigenes, and complementarity-determining regions to binding of antibody-combining sites were analyzed (Kabat et al., J. immunol. 1991; 147 ; 1709-1719). Heavy and light chain constructs were co-transfected into CHO cells. Antibodies were purified using protein A affinity chromatography (GE Healthcare).

### Antibody binding assays analyzed by flow cytometry

Flow cytometry experiments. Briefly, 1.10⁵ cells per 96 wells are incubated with a dilution of unconjugated MAb at 10 µg/mL then diluted at 1/10. Unbound antibodies were washed away with PBS (Invitrogen, Villebon sur Yvette, France) supplemented by 1% Bovine Serum Albumin (Sigma, St Quentin Fallavier, France). Subsequently, cells are centrifuged (5 min at 400 g) and bound antibody is detected with a goat polyclonal F(ab')₂ fragment anti-human IgG RPE conjugated (Sigma, Saint Quentin Fallavier, France) at 4°C for 30 min. Detection reagent is washed away and cells are centrifuged (5 min at 400 g) and suspended in 300 µL PBS. Bound detection antibody is quantified on a FACSCAN (BD Biosciences, Rungis, France), (FL2 channel, 2 000 events per acquisition). During the experiment, the respective isotype controls are included to exclude any unspecific binding events.

### MAb affinity determination

To evaluate binding to DR5, GAH was immobilized to a Biacore sensor Chip CM5 (Biacore-GE Healthcare, France) according to the manufacturer instructions. Carboxyl groups on the sensor Chip surface were activated by injected mix of EDC and NHS. GAH (100µg/mL) in acetate buffer (pH 5.0) was injected over the activated Chip surface. Excess surface reactive groups were deactivated by injecting Ethanolamine. The final level of immobilized MAb was approximately 7000 Resonance Units. A blank, mock-coupled reference surface was prepared on the sensor Chip for background substraction. MAb variant anti-DR5 (5µg/mL) was injected over the immobilized GAM surface. Purified human sDR5 was injected at variable concentration (40nM to 2.5nM). After a 6 minutes dissociation, each surface was regenerated by injecting 10mM glycine pH 1.5 for 30s (2 cycles). Kinetic sensorgram analysis was performed using BIA evaluation software to determine the binding affinity (KD).

### In vitro Biologic MAb activity related to cell proliferation

Biochemical reagents. Biochemical reagents used for the cell proliferation related studies were: recombinant TRAIL (R&D Systems, Lille, France) and Cell Titer GLo-ATP (Promega, Charbonnières-les-bains, France).

Cell viability analysis following ATP level determination. The CellTiter-Glo® Luminescent Cell Viability Assay (Promega, Charbonnières les Bains, France) was used to determine the number of viable cells in culture based on quantification of the ATP present, an indicator of metabolically active cells. Detection is based on using the luciferase reaction to measure the amount of ATP from viable cells. Within minutes after a loss of membrane integrity, cells lose the ability to synthesize ATP, and endogenous ATPases destroy any remaining ATP; thus the levels of ATP fall precipitously. Cell cultures (5.10⁴ cells/mL) are incubated for 72 hours alone, with isotype related MAb control (CTRL-MAb), with positive control MAb (CTRL+ MAb) or with the different anti-DR5 MAb variant alone or with some MAb anti DR5 combination MAb. The CellTiter-Glo^{®} reagent was added directly to cells in culture at a ratio of 50µL of reagent to 200µL of culture medium. The assay plates are incubated at room temperature for 10 min and the bioluminescent signal is recorded using a standard multiwell fluorometer Mithras LB940, (Berthold, Thoiry, France).

### In vitro Biologic MAb activity related to cell apoptosis

Biochemical reagents. Biochemical reagents used for the apoptosis studies were: recombinant TRAIL (R&D Systems, Lille, France) and Caspase 3/7 assay (Promega, Charbonnières-les-bains, France).

Cleaved caspase-3/ quantification for measuring MAb induced apoptosis. Cytotoxicity was evaluated using the Caspase-Glo assays. Briefly, cells were plated at 1.10⁴ cells / wells in 96-well micro titer plates and treated with 100µL fresh medium containing MAb cultured for 4, 15 or 24 hours. The specific Caspase-Glo^{®} (3/7 or 8 or 9) reagent was added directly to cells in culture at a ratio of 50µL of reagent to 100µL of culture medium. The assay plates were incubated at room temperature for 30 min and the bioluminescent signal recorded using a standard multiwell fluorometer Mithras LB940, (Berthold, Thoiry, France). The effects of MAb treatment were expressed in RLU using untreated cells as the RLU control.

### In vitro biologic MAb activity related to Antibody dependent cell cytotoxicity.

Antibody dependent cell cytoxicity Assay (ADCC): Target cells such as the B-cell line Raji (CD19+DR5+) or the Glioma cell line H4 (CD19-DR5+) were loaded with 12.5 µM Calcein-AM dye (Sigma, France). 5 000 target cells per well were the pre-incubated with different concentration of interest MAbs and controls for 20 min at +4° C. 50µL of human whole blood used as effector cells were then added to the target cells. Specific ADCC lysis was calculated using the formula above: (experimental release - (spontaneous release Target + Effector)) / (maximal release-spontaneous release target) * 100, where target and effector cells without antibody represented spontaneous release. Maximal release value was obtained by treating target cells with Triton X-100.

### In vitro biologic MAb activity related to Complement dependent cell cytotoxicity

Complement dependent cytoxicity Assay (CDC): Cells such as the B-cell line Raji (CD19+DR5+) or the Glioma cell line H4 (CD19-DR5+) were incubated with various MAbs concentration. Then, human normal serum were added the culture and then cells were incubated 24 hours at +37° C. At the end of incubation, lactate deshydrogenase present in supernatant was measured with LDH assay kit (Promega, France). Fluorescence was recorded with an excitation wavelength of 560nm and an emission wavelength of 590nM.. Specific CDC lysis was calculated using the formula above: (experimental release-target spontaneous release) / (maximal release-target spontaneous release)*100, where serum and target cells without antibody represented spontaneous release. Maximal release value was obtained by treating target cells with Triton X-100.

### Glycosylation analysis:

Release of *N*-glycans & permethylation were carried out following standard procedures (Ciucanu *et al.,* 1984). IgG were denatured in 0.5% sodium dodecyl sulfate (SDS) and 1% β-mercaptoethanol (+90°C, 5 min) and deglycosylated by enzymatic digestion 15 hours with PNGase F (ROCHE) at +37°C in phosphate buffer, pH 7.5 (Morelle *et al.,* 2007). N-glycans were purified on Ultra Clean SPE Carbograph (ALLTECH). After elution with 25 % acetonitrile, 0.1% TFA, the *N*-glycans were lyophilisated before permethylation. Permethylation using sodium hydroxide procedure (Ciucanu *et al.,* 1984) was performed. After derivatization, the reaction products were purified on C18 Sep Pak Plus (WATERS) and lyophilisated before MALDI TOF MS. Analysis of protein glycosylation was determined by mass spectrometry (Morelle *et al.,* 2007). The purified permethylated *N*-glycans were solubilised with 20 µl of 50% methanol. 1 µl was mixed with 1µl of 2,5 DHB (LaserBiolabs) matrix solution (10 mg /ml, 50% methanol). Positive ion reflectron MALDI mass spectra were acquired on a MALDI TOF MS VOYAGER DE PRO (Applied Biosystems). The spectra were obtained by accumulation of 500 shots and were calibrated with an external standard (LaserBiolabs). The acceleration and reflector voltage conditions were set as follows: voltage 20kV, grid 75%, guide wire 0.002% and delay time 175 ns. MALDI-PSD fragmentation was performed on selected ions using the following conditions: target voltage 20kV, first grid 80% of target voltage, delayed extraction 125ns. Interpretation of glycan structures corresponding to monoisotopic masses was performed using EXPAZY GlycoMod tool.

### RESULTS

### Generation of MAb variants

As used herein, the term "heavy chain" is used to define the heavy chain of an IgG antibody. In an intact, native IgG, the heavy chain comprises the immunoglobulin domains VH, CH1, Hinge, CH2 and CH3. Throughout the examples, the numbering of the residues in an IgG heavy chain is that of the EU index as in Kabat *et al,* (1991), expressly incorporated herein by references. The "EU index as in Kabat®" refers to the numbering of the human IgG1 EU antibody.

We constructed several Fc variants including single, double, three, four, six or seven amino acid substitutions as presented in Table 6.

| **Table 6** | **MAb Fc Variant** |
|---|---|
| Mut0 | Native IgG ref IMGT IpHG1*01 |
| Mut1 | E333A |
| Mut2 | K326A |
| Mut3 | P396L |
| Mut4 | K326A E333A |
| Mut5 | K326A P396L |
| Mut6 | E333A P396L |
| Mut7 | K326A E333A P396L |
| Mut8 | Y300L K326A E333A P396L |
| Mut9 | F243L R292P Y300L K326A E333A P396L |
| Mut10 | R292P Y300L V305L K326A E333A P396L |
| Mut11 | F243L R292P Y300L V305L K326A E333A P396L |

Several humanized anti-DR5 MAb were prepared and evaluated in the present study. Table 7 shows the codification and the VH and VL sequence source of these different anti-DR5 MAb:

| **Codification** | **Sequence source** |
|---|---|
| Hz033 | SEQ ID NO : 2 and 4 |
| Hz031 | SEQ ID NO : 6 and 8 |
| R009E-C | Ref IMGT INN9029 |
| R009E-D | Ref IMGT INN9255 |
| R009E-L | Ref IMGT INN8753 |
| R009E-T | Ref IMGT INN8979 |
| R009E-Y | WO2008066854 |

Variants of these MAb were prepared as it will appear from the following description, wherein these variants comprise an Fc variant as disclosed in Table 6. By convention, the name of the variant MAb makes reference to the Fc variant Mutx, with x = 1 to 11. Mut0 simply indicates native Fc (no mutation).

**Cell staining with MAb variants**

The MAb cellular staining was investigated on H4 positive DR5 cell line (representative data on two independent experiments). Table 8 shows as for example the cell staining with MAb concentration at 10, 1 or 0.1 µg/mL. The percentage of labelled cells (%) and the staining intensity (Mean Fluorescence Intensity) was determined. No significant variation was observed among the mutated MAb variant panel tested compared to the native MAb (Mut0) derivate from the humanized MAb anti-DR5 called Hz031 or Hz033 (as disclosed in WO 2014009358). Similar cellular pattern was observed.

Table 8 shows the FACS analysis of H4 cell staining with MAb variant panel related to Hz031 or Hz033 MAb anti-DR5 tested at different concentrations (percentage of labelled cells %, mean fluorescence intensity / MFI):

| **MAb Hz031 variant** | 10µg/ml | | 1µg/ml | | 0,1µg/ml | |
|---|---|---|---|---|---|---|
| | % | MFI | % | MFI | % | MFI |
| Mut0 | 88 | 209 | 93 | 220 | 75 | 185 |
| Mut1 | 85 | 203 | 90 | 216 | 60 | 169 |
| Mut2 | 89 | 210 | 91 | 217 | 66 | 177 |
| Mut3 | 84 | 200 | 90 | 216 | 78 | 190 |
| Mut4 | 81 | 197 | 91 | 217 | 70 | 182 |
| Mut5 | 90 | 211 | 93 | 219 | 69 | 179 |
| Mut6 | 84 | 201 | 93 | 221 | 77 | 189 |
| Mut7 | 79 | 191 | 90 | 213 | 58 | 166 |
| | | | | | | |

| **MAb Hz033 variant** | 10µg/ml | | 1µg/ml | | 0,1µg/ml | |
|---|---|---|---|---|---|---|
| | % | MFI | % | MFI | % | MFI |
| Mut0 | 87 | 246 | 84 | 235 | 78 | 223 |
| Mut1 | 85 | 238 | 78 | 225 | 70 | 213 |
| Mut2 | 81 | 230 | 80 | 224 | 57 | 196 |
| Mut3 | 82 | 230 | 78 | 223 | 71 | 214 |
| Mut4 | 86 | 239 | 80 | 226 | 56 | 195 |
| Mut5 | 81 | 230 | 79 | 226 | 65 | 206 |
| Mut6 | 83 | 237 | 78 | 225 | 64 | 204 |
| Mut7 | 84 | 237 | 76 | 222 | 65 | 204 |

### Affinity of MAb variants

K_{D} value based on association and dissociation rate constants: Table 9:

| **Hz031 MAb related variant** | **Mutation** | **KD (M)** |
|---|---|---|
| Mut0 | Native IgG | 4,35E-10 |
| Mut7 | K326A E333A P396L | 4,05E-10 |
| Mut8 | Y300L K326A E333A P396L | 4,82E-10 |
| Mut11 | F243L R292P Y300L V305L K326A E333A P396L | 2,84E-10 |

| **Hz033 MAb related variant** | **Mutation** | **KD (M)** |
|---|---|---|
| Mut0 | Native IgG | 2,76E-10 |
| Mut7 | K326A E333A P396L | 4,26E-10 |
| Mut8 | Y300L K326A E333A P396L | 4,00E-10 |
| Mut11 | F243L R292P Y300L V305L K326A E333A P396L | 4,64E-10 |

Whatever the MAb variant tested, similar K_{D} ranges compared to the native MAb were observed such around 2.84 to 4.82 E⁻¹⁰ M for the humanized MAb variants derivate from the Hz031 MAb and around 2.76 to 4.64 E⁻¹⁰ M for the for the humanized MAb variants derivate from the Hz033 MAb.

### In vitro Biologic MAb activity related to cell proliferation

This example illustrates methods of evaluating MAb impact on their ability to trigger cellular cytotoxic effect on DR5 positive cancer cells. These components may be assayed for anti-tumor activity, using any of a number of suitable assays, including but not limited to assays for the ability to slow tumor growth or to kill cancer cells *in vitro.* Various tumor-derived cell lines are among the target cells that may be contacted with MAb combination, in such assay procedures.

To identify MAb anti-DR5 impact on cell viability, the tumor growth is assessed by ATP (Figure 1 to Figure 6)

Results of experiments to determine the agonist activity of the mutated variant MAb derivate from the humanized Hz031 or Hz033 anti-DR5 MAb are shown in Figure 1 or in Figure 2. Different inhibition level was observed of inducing cellular cytotoxicity in H4 cells. Interestingly the MAb variants Hz031- and Hz033-Mut7 (K326A E333A P396L) exhibited the highest level of inhibition of H4 cell growth, whereas no improvement was observed from the single MAb variant Hz031- and Hz033-Mut1 (E333A), Mut2 (K326A) or Mut3 (P396L).

MAb variants based on the integration of the mutations K326A E333A P396L were constructed from different MAb anti-DR5 codified as MAb R009E-C, R009E-D, R009E-L, R009E-T or R009E-Y (Table 7). Comparative MAb biological activity between wild type, unmodified (Mut0) and mutated (Mut7) MAb variants was determined (Figure 3) on different DR5 positive glioma cell lines such as A172 (Figure 3A), H4 (Figure 3B) and T98G (Figure 3C). A significant potency improvement with the Mut7 MAb variants to control cell growth was observed as evidence by ATP, (mean +/- SD on 2 independent experiments).

Different Hz033 MAb variants comprising additional mutations with respect to Hz033-Mut7 were also generated: Hz033-Mut8 (Y300L K326A E333A P396L), Hz033-Mut9 (F243L R292P Y300L K326A E333A P396L), Hz033-Mut10 (R292P Y300L V305L K326A E333A P396L) and Hz033-Mut11 (F243L R292P Y300L V305L K326A E333A P396L). Then their ability to control H4 cell growth was determined as evidence by ATP, (mean +/- SD on 2 independent experiments), (Figure 4).No significant improvement of the inhibition level on H4 cell growth compared to the Hz033-Mut7 MAb variant (K326A E333A P396L substitutions) was found..

We previously demonstrated that the Hz031 and H033 MAb combination exhibited a higher control on cell proliferation (WO 2014009358). The impact on the K326A E333A P396L substitutions were tested herein on the cell growth as evidence by ATP quantification from GBM cell lines exhibiting different sensitivity level of TRAIL induced apoptosis (Figure 5), (mean +/- SD on 2 independent experiments). As shown in Figure 6, a significant MAb potency improvement of the MAb anti-DR5 Hz031 and H033 combination following the K326A E333A P396L substitutions was observed, (Mean +/- SD on 2 independent experiments). The antibody of the invention allows to reverse apoptosis resistance of highly or a little apoptosis resistant DR5-expressing cells, which means that the antibody is efficient in inducing apoptosis of these cells whereas the wild-type antibodies is not.

### In vitro Biologic MAb activity related to cell apoptosis

When apoptosis is activated, caspases cleave multiple protein substrates, which leads to the loss of cellular structure and function, and ultimately results in cell death. In particular, caspases -8, -9, and -3 have been implicated in apoptosis: caspase-9 in the mitochondrial pathway, caspase-8 in the Fas/CD95 pathway, and caspase-3 more downstream, activated by multiple pathways. As shown in Figure 7, the MAb anti-DR5 Hz031 and H033 combination following the K326A E333A P396L substitutions (Mut7) exhibited a higher apoptotic activity compared to the Mut0 MAb combination in H4 cell line as evidence by cleaved caspase 3/7 quantification, (Mean+/- SD on 2 independent experiments).

### In vitro Biologic MAb activity related to ADCC or CDC

In many applications, antibodies have demonstrated improved effector function in complement-mediated tumor cells lysis and in antibody-dependent cellular cytotoxicity assays.

The MAb activity to mediate ADCC from the MAb variant panel was measured using H4 target cells in whole blood based assays (Figure 8). Only the MAb variant with the Mut8 integration (Y300L K326A E333A P396L) or Mut11 integration (F243L R292P Y300L V305L K326A E333A P396L) on the MAb anti-DR5 Hz031 and H033 combination exhibited a higher level ADCC activity compared to the native (Mut0). No significant Mut7 integration (K326A E333A P396L) impact compared to the native (Mut0) on the MAb anti-DR5 Hz031 and H033 combination was observed. These data suggest no correlation between Mut7 related amino acid sequences and ADCC potency.

The MAb activity to mediate CDC from the MAb variant panel was measured using H4 target cells (Figure 9). No complement dependent cytotoxicity improvement was observed following the Mut7, Mut8 or Mut11 integration compared to the native (Mut0) on the MAb anti-DR5 Hz031 and H033 combination was observed. These data suggest no strong correlation between CDC potency and those amino acid sequences.

### Characterization of the glycosylation pattern of MAb variants

The sugar core found in the Fc region of IgG is a bi-antennary complex [Asn297-GN-GN-M-(M-GN)2] where GN is N-acetylglucosamine, and M is mannose. Oligosaccharides can contain zero (G0), one (G1) or two (G2) galactose (G). Variations of IgG glycosylation patterns can include core fucosylation (F). As shown in Figure 10, similar glycan profiles was observed with the Mut7 (K326A E333A P396L) or Mut8 (Y300L K326A E333A P396L) derivate Hz031 compared to the native (Mut0) Hz031 MAb corresponding to masses of fucosylated oligosaccharides with (GlcNAc)2 (Fuc)1 + (Man)3 (GlcNAc)2 (m/z 1836), (Gal)1 (GlcNAc)2 (Fuc)1 + (Man)3(GlcNAc)2 (m/z 2040) and (Gal)2 (GlcNAc)2 (Fuc)1 + (Man)3(GlcNAc)2 (m/z 2245). Other typical structure of N-linked oligosacchararide on IgG was also observed, characterized by a mannosyl-chitobiose core (Man3-GlcNac2-Asn) with or without bisecting GlcNac/L-Fucose (Fuc) and other chain variants including the presence or absence of Galactose (Gal) and sialic acid. In addition, oligosaccharides may contain zero (GO), one (GI) or two (G2) Gal. Therefore no structural difference compared to the native MAb was observed following the K326A E333A P396L substitutions (Mut7) or following the Y300L K326A E333A P396L substitutions (Mut8). By contrast a significant structural variation was observed following the F243L R292P Y300L V305L K326A E333A and P396L substitutions (Mut11) as previously described in WO2012/010602.

To help interpretation of Figure 10, Table 10 shows molecular mass of permethylated glycans, detected as [M+Na]+ by MALDI mass spectrometry of Hz031 MAb anti DR5 MAb related to native (Mut0), Mut7 (K326A E333A P396L) Mut8 (Y300L K326A E333A P396L) or Mut11 (F243L R292P Y300L V305L K326A E333A P396L):

| **Theoretical mass** | **Interpretation** |
|---|---|
| **[M+Na]⁺ permeth** | |
| 1416,71 | (GlcNAc)₁ + (Man)₃(GlcNAc)₂ |
| 1579,78 | (Man)₅(GlcNAc)₂ |
| 1590,84 | (GlcNAc)₁ **(Fuc)₁** + (Man)₃(GlcNAc)₂ |
| 1620,81 | (GlcNAc)₁ + (Man)₄(GlcNAc)2 |
| 1661,84 | (GlcNAc)₂ + (Man)₃(GlcNAc)₂ |
| 1783,80 | (Man)₆(GlcNAc)₂ |
| 1794,90 | (Gal)₁ (GlcNAc)₁ **(Fuc)₁** + (Man)₃(GlcNAc)₂ |
| 1824,92 | (GlcNAc)₁ + (Man)₅(GlcNAc)₂ |
| 1835,93 | (GlcNAc)₂ **(Fuc)₁** + (Man)₃(GlcNAc)₂ |
| 1865,94 | (GlcNAc)₂ **(Fuc)₁** + (Man)₃(GlcNAc)₂ hypermeth* or (Gal)₁ (GlcNAc)₂ + (Man)3(GlcNAc)2 |
| 1987,98 | (Man)₇(GlcNAc)₂ |
| 2040,03 | (Gal)₁ (GlcNAc)₂ **(Fuc)₁** + (Man)₃(GlcNAc)₂ |
| 2192,09 | (Man)₈(GlcNAc)₂ |
| 2244,12 | (Gal)₂ (GlcNAc)₂ **(Fuc)₁** + (Man)₃(GlcNAc)₂ |
| 2396,18 | (Man)₉(GlcNAc)₂ |

## Claims

1. An anti-DR5 antibody comprising an immunoglobulin domain binding specifically to a DR5 receptor and a variant human IgG Fc region, preferably IgG1 Fc region, wherein the variant IgG Fc region comprises an amino acid substitution at the amino acid positions 326 and 333 ; 326 and 396 ; 333 and 396; or 326, 333 and 396.

2. The antibody of claim 1, wherein the amino acid positions are substituted as follows: 326A, 333A and/or 396L.

3. The antibody of claim 1, wherein the variant IgG Fc region has substitution K326A, E333A and P396L.

4. The antibody of claim 1, comprising a variant IgG1 Fc region comprising substitutions K326, E333 and P396 or which comprises solely substitutions at K326, E333 and P396.

5. The antibody of claim 1, comprising a variant IgG Fc region comprising the following amino acid substitutions : Y300L K326A E333A P396L ; F243L R292P Y300L K326A E333A P396L ; R292P Y300L V305L K326A E333A P396L ; or F243L R292P Y300L V305L K326A E333A P396L.

6. The antibody of any one of claims 1-5, comprising a Fab region comprising a pair of VH and VL chains wherein :
- the VH chain contains a CDR1 comprising or consisting of sequence SEQ ID NO: 13, a CDR2 comprising or consisting of sequence SEQ ID NO: 14, a CDR3 comprising or consisting of sequence SEQ ID NO: 15; and the VL chain contains a CDR1 comprising or consisting of sequence SEQ ID NO: 16, a CDR2 comprising or consisting of sequence FAS, a CDR3 comprising or consisting of sequence SEQ ID NO: 17 ;
- the VH chain contains a CDR1 comprising or consisting of sequence SEQ ID NO:22, a CDR2 comprising or consisting of sequence SEQ ID NO: 23, a CDR3 comprising or consisting of sequence SEQ ID NO: 24; and the VL chain contains a CDR1 comprising or consisting of sequence SEQ ID NO: 25, a CDR2 comprising or consisting of sequence SEQ ID NO: 26, a CDR3 comprising or consisting of sequence SEQ ID NO: 17 ;
- the VH chain contains a CDR1 comprising or consisting of sequence SEQ ID NO : 32, a CDR2 comprising or consisting of sequence SEQ ID NO: 14, a CDR3 comprising or consisting of sequence SEQ ID NO: 24; and the VL chain contains a CDR1 comprising or consisting of sequence SEQ ID NO: 16, a CDR2 comprising or consisting of sequence FAS, a CDR3 comprising or consisting of sequence SEQ ID NO: 17 ;
- the VH chain contains a CDR1 comprising or consisting of sequence SEQ ID NO: 18, a CDR2 comprising or consisting of sequence SEQ ID NO: 14, a CDR3 comprising or consisting of sequence SEQ ID NO: 19; and the VL chain contains a CDR1 comprising or consisting of sequence SEQ ID NO: 20, a CDR2 comprising or consisting of sequence RTS, a CDR3 comprising or consisting of sequence SEQ ID NO: 21 ;
- the VH chain contains a CDR1 comprising or consisting of sequence SEQ ID NO: 27, a CDR2 comprising or consisting of sequence SEQ ID NO: 28, a CDR3 comprising or consisting of sequence SEQ ID NO: 29; and the VL chain contains a CDR1 comprising or consisting of sequence SEQ ID NO: 30, a CDR2 comprising or consisting of sequence SEQ ID NO: 31, a CDR3 comprising or consisting of sequence SEQ ID NO: 21 ; or
- the VH chain contains a CDR1 comprising or consisting of sequence SEQ ID NO : 33, a CDR2 comprising or consisting of sequence SEQ ID NO: 14, a CDR3 comprising or consisting of sequence SEQ ID NO: 29; and the VL chain contains a CDR1 comprising or consisting of sequence SEQ ID NO: 20, a CDR2 comprising or consisting of sequence RTS, a CDR3 comprising or consisting of sequence SEQ ID NO: 21.

7. The antibody according to claim 6, comprising one or more of amino acid sequence pairs SEQ ID NO : 2 and 4 and SEQ ID NO : 6 and 8, the pair of amino acid sequences SEQ ID NO : 2 and 4, the pair of amino acid sequences SEQ ID NO : 6 and 8, or both amino acid sequence pairs SEQ ID NO : 2 and 4 and SEQ ID NO : 6 and 8.

8. The antibody according to any one of claims 1-7, as a medicament.

9. The antibody according to any one of claims 1-8, for use in inducing apoptosis of cells expressing DR5.

10. The antibody for the use according to claim 9, wherein the antibody has a variant IgG Fc region comprising amino acid substitutions at the amino acid positions 326 and 333 ; 326 and 396 ; 333 and 396; or 326, 333 and 396, and does not induce ADCC and/or does not regulate CDC and/or have no altered glycan profile.

11. The antibody for the use according to claim 9, wherein the antibody has a variant IgG Fc region comprising amino acid substitutions at the amino acid positions 326 and 333 ; 326 and 396 ; 333 and 396; or 326, 333 and 396, and at least one additional mutation and the antibody has ADCC and/or an altered glycan profile dues to this additional mutation.

12. The antibody for the use according to claim 9, 10 or 11, wherein the antibody induces apoptosis of cells expressing DR5 that are resistant to apoptosis.

13. An antibody according to any one of claims 1-8, for use as an anti-cancerous agent.

14. An antibody according to any one of claims 1-8, for use for treating an autoimmune disease, an inflammatory disease or condition, an infectious disease such as a viral infection or viral disease, a neurological disorder.

15. An antibody for the use of claim 13 or 14, which induces apoptosis of cells having a DR5 receptor.
